# EUROPEAN PATENT SPECIFICATION

(11) **EP 3 592 729 B1**
(45) Date of publication and mention of the grant of the patent: **30.06.2021**
(21) Application number: 18709992.4
(22) Date of filing: 04.03.2018
(51) Int. Cl.: C07C 391/02, C07C 319/06, C07D 241/18, C07D 417/06, C07D 277/74, C07D 213/32, C07C 321/28, C07C 321/22, C07C 323/20, C07C 323/35, C07C 323/62, C07F 7/08, C07F 7/18, C07F 9/50, C07J 31/00

(54) **CATALYTIC C-X-BOND METATHESIS THROUGH ARYLATION**
KATALYTISCHE C-X-GEBUNDENE METATHESE DURCH ARYLIERUNG
MÉTATHÈSE DE LIAISON C-X CATALYTIQUE PAR ARYLATION

(30) Priority: 07.03.2017 DE 102017203746
(43) Date of publication of application: 15.01.2020
(73) Proprietor: Studiengesellschaft Kohle mbH, 45470 Mülheim (DE)
(72) Inventor: LIAN, Zhong, Chengdu Wuhou District 610044 (CN); MORANDI, Bill, Uetikon am See 8707 Zurich (CH); BHAWAL, Benjamin N., 8050 Zurich (DE); YU, Peng, 08540 New Jersey (US); DELCAILLAU, Tristan, 8050 Zurich (CH)
(86) International application number: PCT/EP2018/055238
(87) International publication number: WO 2018/162364

(56) References cited:
- MIEKO ARISAWA ET AL: "Transition-metal-catalyzed synthesis of organosulfur compounds", PURE & APPLIED CHEMISTRY, vol. 80, no. 5, 1 January 2008 (2008-01-01), pages 993-1003, XP55471611, GB ISSN: 0033-4545, DOI: 10.1351/pac200880050993
- CHRISTEN?M. BELL ET AL: "Catalytic Metathesis of Simple Secondary Amides", ANGEWANDTE CHEMIE INTERNATIONAL EDITION, vol. 46, no. 5, 22 January 2007 (2007-01-22), pages 761-763, XP55471609, ISSN: 1433-7851, DOI: 10.1002/anie.200603588

## Description

The present invention refers to a process for a catalytic aryl transfer to rearrange the backbone of aromatic C-X bonds.

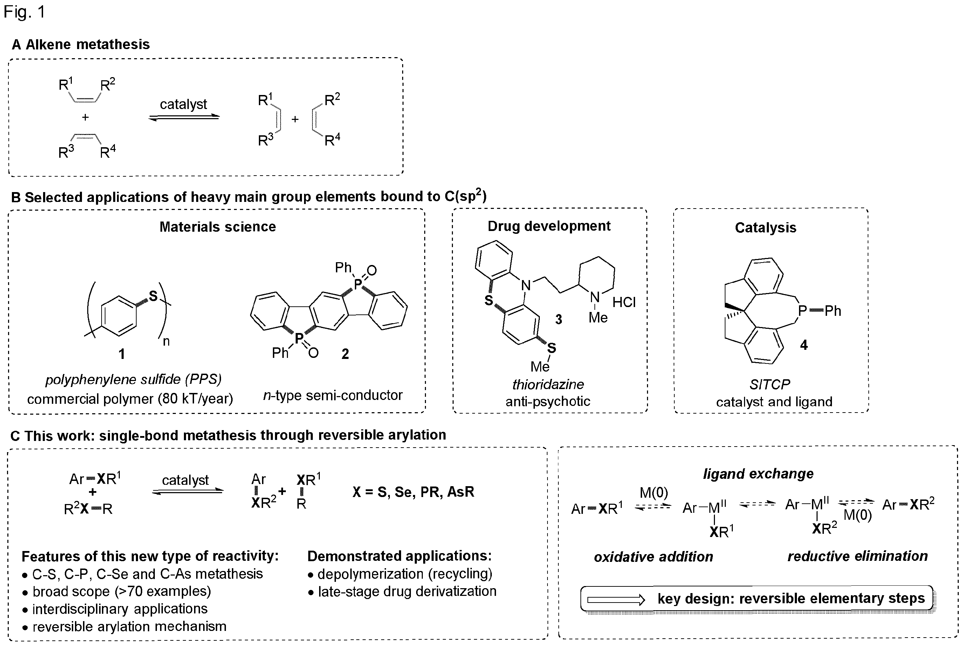

The alkene metathesis reaction has had a transformative impact on chemistry by offering an alternative approach to olefin synthesis that is complementary in scope and reactivity to traditional olefination reactions, such as the Wittig reaction. Due to its versatility, alkene metathesis has consequently found applications in very diverse areas, including polymer chemistry, biomass valorization and drug synthesis due to the ubiquity of alkenes as starting materials, synthetic intermediates and final products. The isodesmic nature of the reaction enables the transformation of one alkene into another in a mild process, leading to an overall exchange of the alkene group substituents (Figure 1A). This feature facilitates the rapid generation of new molecular architectures while conserving the important olefin functionality. In light of the established synthetic power of alkene metathesis, it can be expected that the metathesis of other important bonds including single bonds would have a beneficial impact on the molecular sciences.

Carbon-heteroatom bonds composed of heavy main group elements are commonly encountered in a wide range of applications (Figure 1B). In particular, C(sp²)-S and C(sp²)-P bonds are essential in materials and medicinal sciences. Aromatic thioethers are key motifs in drug development and can also be found in many organic materials and polymers - for example, the thermoplastic polyphenylene sulfide (PPS, **1**) is produced yearly on a 80,000 ton scale. Aromatic phosphines are commonly used as ligands and catalysts, both on laboratory scale and industrial processes. They are further employed in the area of organic materials, with applications ranging from sensors to organic light emitting diodes.

Transition metal-catalysed synthesis of organosulfur compounds is taught in PURE & APPL. CHEMISTRY, vol. 80, no. 5, 2008, pages 993-1003).

There are rare examples of single C-X bond metathesis only, including transamidation processes. The metathesis of aromatic C-X bonds, however, has been virtually unexplored.

It is an object of the present invention therefore to provide a carbon-heteroatom bond metathesis reactions, wherein compounds containing at least one C(sp²)-heteroatom bond could effectively swap their substituents in a manner analogous to alkene metathesis. The challenge in developing catalytic metathesis reactions employing C-X bonds is to identify a mechanistic pathway in which the breakage of a C-X bond and subsequent ligand exchange can be realized. The reaction conditions wherein the oxidative addition and reductive elimination of common C-X bonds, including C-S or C-P bonds, and exchange of the resulting thiolate or phosphine ligand could be achieved to unlock novel catalytic C-X bond metathesis reactions should be identified. It is also an object to identify a general mechanistic manifold, proceeding through transfer arylation, to perform unprecedented catalytic C(sp²)-X bond metathesis with several different main group elements (Figure 1C).

It has now been found that the above-mentioned disadvantages can be dealt with by a process for a catalytic aryl transfer wherein an aryl-compound (I) is reacted with an hydrocarbon (II) in the presence of a Pd- or Ni-catalyst coordinated by electron rich ligands and in the presence of a base in an organic solvent, as represented in the following reaction scheme: 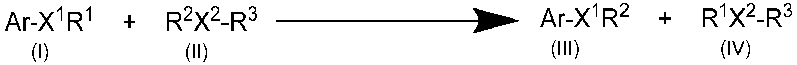 wherein
- Ar is aryl, heteroaryl or vinyl, each being optionally substituted by one or more groups selected from straight chain or branched chain alkyl, cycloalkyl, heterocycloalkyl, alkenyl, alkynyl, aryl, aralkyl, heteroaryl, heteroaralkyl, ether, acetal, silyl ether or amine, or by a heterosubstituent;
- X¹ and X² may be the same or different and are each S or Se, preferably S,
- R¹ is H or methyl, a straight chain or branched C₂-C₁₆-alkyl, or aryl, each optionally being substituted by one or more groups selected from straight chain or branched chain alkyl, cycloalkyl, heterocycloalkyl, alkenyl, alkynyl, aryl, aralkyl, heteroaryl, heteroaralkyl, ether, acetal, silyl ether or amine, or by a heterosubstituent;
- R² is an primary, secondary or tertiary alkyl or aryl, each being optionally being substituted by one or more groups selected from straight chain or branched chain alkyl, cycloalkyl, heterocycloalkyl, alkenyl, alkynyl, aryl, aralkyl, heteroaryl, heteroaralkyl, or by a heterosubstituent;
- R³ is H,
- the Pd- or Ni-catalyst coordinated by electron rich ligands is selected from the group consisting of Pd(OAc)₂, Pd₂(dba)₃, PdCl₂, PdCl₂(MeCN)₂, Ni(COD)₂.
- the electron rich ligands is selected from the group consisting of IPENT(1,3-Bis(2,6-bis(1-ethylpropyl)phenyl)imidazol-2-ylidene), SIPr (1,3-Bis(2,6-diisopropylphenyl)imidazolidene), ICy (1,3-bis-(cyclohexyl)imidazol-2-ylidene), or IPr (1,3-bis(2,6-diisopropylphenyl)imidazol-2-ylidene).
- the base is selected from the group consisting of LiHMDS, KHMDS, NaHDMS, LiO*t*Bu, KO*t*Bu, NaO*t*Bu.

Such a process represents a powerful companion to traditional cross-coupling processes. This strategy is particularly useful for the rapid discovery and derivatization of functional molecules to prepare compound libraries that are essential for structure activity relationship (SAR) studies. Additionally, the process parallel the alkene metathesis reaction in providing an extremely flexible tool for the construction and deconstruction of organic molecules with potential applications in waste recycling and polymerization.

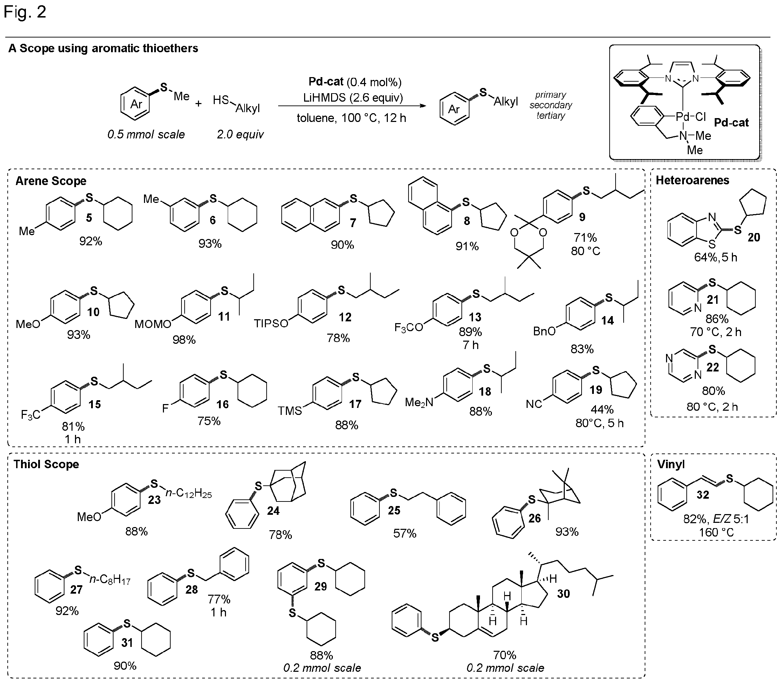

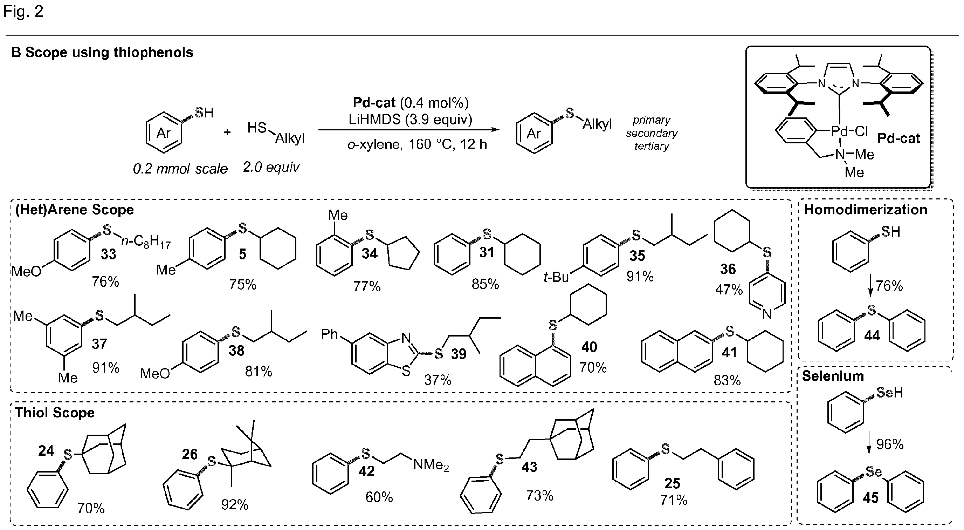

A further attractive feature of this reaction is the possibility, at higher temperatures, to directly use, for example, an unprotected thiophenol as an electrophile through C-S bond cleavage (Figure 2B). This is notable, since previous examples of such reactivity are extremely rare due to catalyst poisoning, and only two thiophenol substrates have been reported to undergo C-S bond cleavage, using Grignard reagents under Ni-catalysis. Homodimerization of thiophenol, which could potentially have plagued the efficiency of a chemoselective coupling process, does not interfere with the inventive reaction when a more nucleophilic alkyl thiol is employed. However, if no cross-metathesis partner is present in the reaction mixture, homodimerization of thiophenol leads to the formation of diphenyl sulfide (44). Another chalcogen element, selenium, can also participate in this reaction and the homodimerization of selenophenol gave high yields of the corresponding diphenylselenide (45).

In one embodiment, a Pd-NHC (N-heterocyclic carbene) complex, preferably a [(NHC)Pd(dimethylbenzylamine)CI] complex is used as catalyst. Such a catalyst efficiently promotes the oxidative addition of Ar-X and its microscopic reverse, reductive elimination, to enable a facile X-R³ group exchange to take place. According to the present invention, the Pd- or Ni- catalyst is present in an amount in the range of of 0.05 mol% to 1 mol% of the aryl-compound (I), preferably in the range of 0.2 mol% to 0.6 mol% of the aryl-compound (I).

In a preferred embodiment, the base used in the process of the present invention is a lithium base, sodium base or potassium base, preferably a lithium base, more preferably LiHMDS (lithium bis(trimethylsilyl)amide). Use of a lithium base proved clearly superior to sodium or potassium bases, reflecting the importance of solubilities in this reaction. The decreased solubility of the side-product, for example MeSLi when Ar-XR¹ is a methyl aromatic thioether, when compared to the larger lithium thiolate salt generated from the thiol reagent and the base, efficiently drives the equilibrium of the reaction to completion.

Preferably, the base is present in an amount in the range of 1 equivalent to 6 equivalents, preferably in the range of 1.5 equivalent to 4 equivalents of the reaction partners.

In a preferred embodiment, the aryl-compound (I) is reacted with the hydrocarbon (II) at a temperature in the range of 25°C to 250°C, preferably in the range of 80°C to 200°C, for 4 h to 20 h, preferably 8 h to 16 h.

The organic solvent used for reacting the aryl-compound (I) with the hydrocarbon (II) is not critical and can be selected amongst those which are commonly used for such kind of catalyzed reactions. Preferably, an aromatic solvent or an aliphatic hydrocarbon solvent, more preferably toluene, benzene, xylene, cumene, chlorobenzene or dichlorobenzene is used.

In a preferred embodiment, Ar is phenyl, 4-methylphenyl or naphtyl, each optionally being substituted by one or more groups selected from straight chain or branched chain alkyl, ether, acetal, silyl ether or amine, preferably being substituted by methyl, ketals, methoxy, MOMO, TIPSO, OCF₃, OBn, CF₃, F, TMS, NMe₂, CN, pyridyl, pyrazinyl, benzothiazyl, phenylvinyl, t-butyl or 5-phenyl-benzothiazyl.

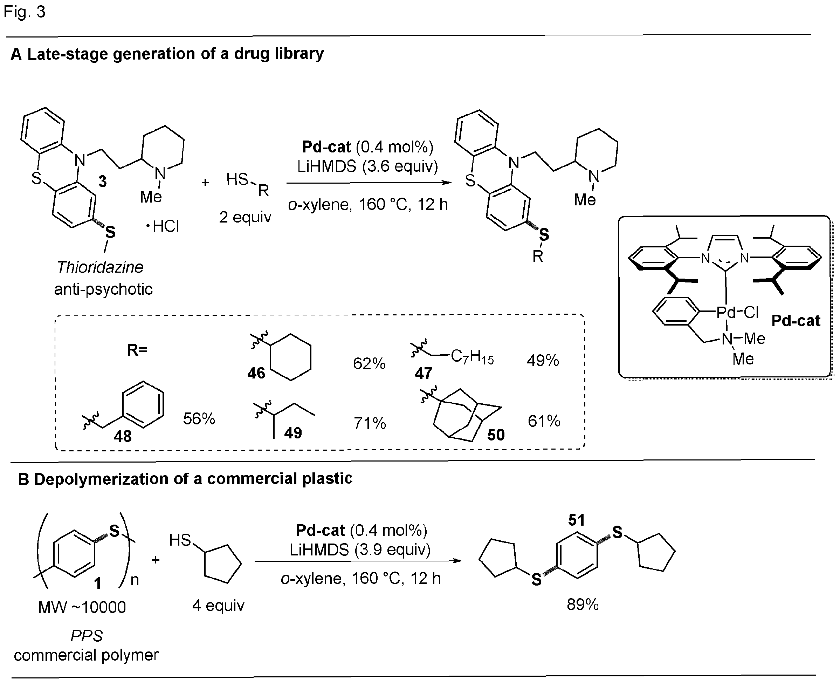

In a preferred embodiment, Ar or Ar-X is a component of a polymer. The process of the present invention provides the possibility to depolymerize polymers, preferably, thermoplastic polymers, in particular PPS (1) to obtain simple chemical building blocks (Figure 3B).

In a preferred embodiment, X is S.

In a further embodiment, R¹ is H, methyl, phenyl or 4-methoxyphenyl.

In one embodiment, R² is cyclohexyl, cyclopentyl, 2-methylbutyl, 1-methyl-propyl, nC₁₂H₂₅, adamantyl, 2-phenylethyl, 1-methyl-5-dimethyl-bicyclo[4.1.0]heptyl-, nC₈H₁₇, benzyl, optionally substituted steroid residue, 2-amantadyl-ethyl or C₈H₁₇.

In a preferred embodiment, the hydrocarbon (II) is present an amount in the range of 0.5 equivalents to 6 equivalents of the aryl-compound (I), preferably in the range of 1.5 equivalent to 4 equivalents of the aryl-compound (I).

In a further embodiment, the process of the present invention is used to enable a sequence of arylation/retro-arylation to take place and equilibrate a simple reaction mixture (Figure 3C). Such a thioether cross-metathesis, in case X is S, leads to the same ratio of starting materials to products in the forward and reverse direction. In such an process, R²X-R³ is used as a co-catalytic amount in the range of 5 to 15%, preferably in the range of 7,5 to 12,5% of the total amount of the at least one Ar-X-R¹ compound.

Accordingly, disclosed herein is also an aryl-compound produced by the process of the present invention.

Definition for the substituents as described herein are given in the following.

A heterosubstituent according to the invention is to be understood as a substituent including heteroatoms, preferentially selected from O, N, S, Si and halogens. It can be preferentially selected from, =O, -OH, -F, -CI, -Br, -I, -CN, -N₃, -NO₂,-SO₃H, NCO, NCS, OP(O)(OR^{S1})(OR^{S2}), OP(OR^{S1})(OR^{S2}), a monohalogenomethyl group, a dihalogenomethyl group, a trihalogenomethyl group, -CF(CF₃)₂, -SF₅,-NR^{S1}₂, -OR^{S1}, -OOR^{S1}, -OSiR^{S1}R^{S2}R^{S3}, -OSi(OR^{S1})R^{S2}R^{S3}, -OSi(OR^{S1})(OR^{S2})R^{S3},-OSi(OR^{S1})(OR^{S2})(OR^{S3}), -OSO₂R^{S1}, -SR^{S1}, -SSR^{S1}, -S(O)R^{S1}, -S(O)₂R^{S1},-C(O)OR^{S1}, -C(O)NR^{S1}R^{S2}, -NR^{S1}C(O)R^{S2}, -C(O)-R^{S1}, -COOM, wherein M may be a metal such as Na, K or Cs.

R^{S1} R^{S2} and R^{S3} each individually represent H, alkyl, cycloalkyl, heterocycloalkyl, aryl, heteroaryl, aralkyl, heteroaralkyl, sulfonyl, silyl, each being optionally substituted by one or more alkyl, cycloalkyl, heterocycloalkyl, heteroaryl, aralkyl, heteroaralkyl, sulfonyl or heterosubstituent.

For the reaction system in more detail, alkyl may be C₁-C₂₀-Alkyl which can be straight chain or branched or cyclic and has 1, 2, 3, 4, 5, 6, 7, 8, 9, 10, 11, 12, 13, 14, 15, 16, 17, 18, 19 or 20 carbon atoms. Alkyl might particularly be C₁-C₆-alkyl, in particular methyl, ethyl, propyl, isopropyl, butyl, isobutyl, sec-butyl or tert-butyl, likewise pentyl, 1-, 2- or 3-methylpropyl, 1,1-, 1,2- or 2,2-dimethylpropyl, 1-ethylpropyl, hexyl, 1-, 2-, 3- or 4-methylpentyl, 1,1-, 1,2-, 1,3-, 2,2-, 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl.

Cycloalkyl may be a cyclic alkyl group forming a 3 to 20 membered ring and might be cyclopropyl, cyclobutyl, cyclopentyl, cyclohexyl, cycloheptyl or cyclooctyl.

Heterocycloalkyl may be a cycloalkyl forming a 3 to 10 membered ring and incorporating one or more heteroatoms selected from N, O and S within the cycle. In particular, heterocycloalkyls can be preferentially selected from 2,3-dihydro-2-,-3-, -4- or -5-furyl, 2,5-dihydro-2-, -3-, -4- or -5-furyl, tetrahydro-2- or -3-furyl, 1,3-dioxolan-4-yl, tetrahydro-2- or -3-thienyl, 2,3-dihydro-1-, -2-, -3-, -4- or -5-pyrrolyl, 2,5-dihydro-1-, -2-, -3-, -4- or -5-pyrrolyl, 1-, 2- or 3-pyrrolidinyl, tetrahydro-1-, -2-or -4-imidazolyl, 2,3-dihydro-1-, -2-, -3-, -4- or -5-pyrazolyl, tetrahydro-1-, -3- or -4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-,-3- or -4-pyranyl, 1,4-dioxanyl, 1,3-dioxan-2-, -4- or -5-yl, hexahydro-1-, -3- or -4-pyridazinyl, hexahydro-1-, -2-, -4- or -5-pyrimidinyl, 1-, 2- or 3-piperazinyl, 1,2,3,4-tetrahydro-1-, -2-, -3-, -4-, -5-, -6-, -7- or -8-quinolyl, 1,2,3,4-tetrahydro-1-, -2-, -3-,-4-, -5-, -6-, -7- or -8-isoquinolyl, 2-, 3-, 5-, 6-, 7- or 8-3,4-dihydro-2H-benzo-1,4-oxazinyl.

Halogen is F, Cl, Br or I.

Aryl might be phenyl, naphthyl or biphenyl and substituted derivatives thereof.

Aralkyl might be benzyl, naphthylmethyl and substituted derivatives thereof.

Heteroaryl may have one or more heteroatoms selected from N, O,S and Si and is preferably 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, also preferably 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or 4-pyridazinyl, pyrazinyl, 1-, 2-, 3-, 4-, 5-, 6- or 7-Indolyl, 4- or 5-isoindolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7- or 8-2H-benzo-1,4-oxazinyl, also preferably 1,3-benzodioxol-5-yl, 1,4-benzodioxan-6-yl, 2,1,3-benzothiadiazol-4- or -5-yl or 2,1,3-benzoxadiazol-5-yl.

Heteroaralkyl might be any of the aforementioned heteroaryl bound to an alkyl group, such as pyridinylmethyl.

Optionally substituted means unsubstituted or monosubstituted, disubstituted, trisubstituted, tetrasubstituted, pentasubstituted, or even further substituted on the respective group.

The invention is further illustrated in the attached figures and the following experimental section below.

In the attached drawings:
Figure 1 illustrates the:
   (A) Alkene metathesis.
   (B) Selected applications of heavy main group elements bound to C(sp²).
   (C) The process of the present invention showing a single-bond metathesis through arylation.
Figure 2 illustrates the:
   (A) Products obtained by the process of the present invention using aromatic thioethers as Ar-XR¹ educt.
   (B) Products obtained by the process of the present invention using thiophenols as Ar-XR¹ educt.
Figure 3 illustrates the synthetic potential of the C-S bond metathesis reaction of the present invention, in particular Figure 3 shows the:
   (A) Late stage generation of a drug library using Thioridazine as Ar-XR¹ starting material.
   (B) Depolymerization of a commercial plastic.

### Experimental Section

### Preparation Examples

### General procedure for the catalytic aryl transfer with aryl ethers

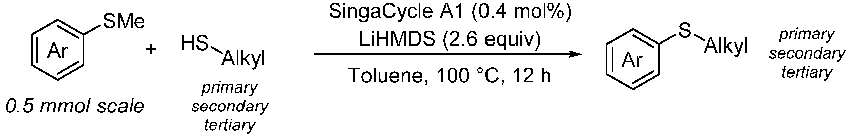

In the glovebox, aryl methyl sulfane (0.5 mmol), alkyl thiol (2.0 equiv, 1.0 mmol), LiHMDS (1.3 ml, 1.0 M in toluene), and SingaCycle A1 (0.4 mol%, 0.4 ml, 0.005 M in toluene) were added into an oven-dried 8 ml vial with a magnetic stirring bar, followed by addition of toluene (0.3 ml). The vial was sealed and removed out of the glovebox and heated to 100 °C. After 12 h, the vial was cooled to room temperature. The reaction was diluted with ethyl acetate and washed with saturated NaOH solution. The aqueous phase was extracted with ethyl acetate 3 times. The collected organic phases were dried over anhydrous Na₂SO₄. The solvent was removed under reduced pressure and the residue was purified by flash column chromatography to give the desired product.

### Example 1

### cyclohexyl(p-tolyl)sulfane

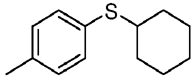

Prepared by general procedure A; isolated as a colorless liquid using pentane/ethyl acetate (100:1) as eluent (94.2 mg, 92%). ¹H NMR (300 MHz, Chloroform-*d*) δ 7.31 (d, *J =* 7.8 Hz, 2H), 7.10 (d, *J =* 7.8 Hz, 2H), 3.12-2.94 (m, 1H), 2.33 (s, 3H), 2.06-1.91 (m, 2H), 1.84-1.71 (m, 2H), 1.66-1.57 (m, 1H), 1.44-1.15 (m, 5H). ¹³C NMR (75 MHz, CDCl₃) δ 136.8, 132.7, 131.2, 129.5, 47.1, 33.4, 26.1, 25.8, 21.0. HRMS C₁₃H₁₈S [M]⁺; calculated 206.1123, found: 206.1126. The spectral data are consistent with those reported in the literature.

### Example 2

### cyclohexyl(m-tolyl)sulfane

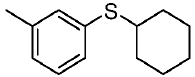

Prepared by general procedure A; isolated as a colorless liquid using pentane/ethyl acetate (100:1) as eluent (95 mg, 93%). ¹H NMR (300 MHz, Chloroform-*d*) δ 7.25-7.12 (m, 3H), 7.03 (s, 1H), 3.23-3.01 (m, 1H), 2.33 (s, 3H), 2.05-1.93 (m, 2H), 1.77 (q, *J =* 5.4, 4.4 Hz, 2H), 1.66-1.58 (m, 1H), 1.45-1.20 (m, 5H). ¹³C NMR (126 MHz, CDCl₃) δ 138.5, 134.9, 132.5, 128.8, 128.6, 127.5, 46.6, 33.4, 26.1, 25.8, 21.3. HRMS C₁₃H₁₈S [M]⁺; calculated 206.1123, found: 206.1125. The spectral data are consistent with those reported in the literature.

### Example 3

### cyclopentyl(naphthalen-2-yl)sulfane

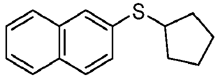

Prepared by general procedure A; isolated as a pale yellow liquid using pentane/ethyl acetate (100:1) as eluent (102.3 mg, 90%). ¹H NMR (300 MHz, Chloroform-*d*) δ 7.87-7.73 (m, 4H), 7.57-7.38 (m, 3H), 3.96-3.65 (m, 1H), 2.24-2.03 (m, 2H), 1.92-1.60 (m, 6H). ¹³C NMR (75 MHz, CDCl₃) δ 134.8, 133.7, 131.7, 128.1, 128.0, 127.64, 127.63, 127.0, 126.4, 125.5, 45.8, 33.5, 24.8. HRMS C₁₅H₁₆S [M]⁺; calculated 228.0967, found: 228.0968.

### Example 4

### cyclopentyl(naphthalen-1-yl)sulfane

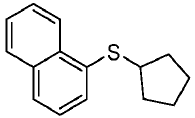

Prepared by general procedure A; isolated as a pale yellow liquid using pentane/ethyl acetate (100:1) as eluent (103 mg, 91%). ¹H NMR (300 MHz, Chloroform-*d*) δ 8.55-8.42 (m, 1H), 7.92-7.82 (m, 1H), 7.81-7.71 (m, 1H), 7.65 (dd, *J* = 7.2, 1.2 Hz, 1H), 7.58-7.52 (m, 2H), 7.43 (dd, *J* = 8.2, 7.2 Hz, 1H), 3.68 (td, *J* = 6.3, 5.7, 1.4 Hz, 1H), 2.19-1.96 (m, 2H), 1.92-1.80 (m, 2H), 1.78-1.58 (m, 4H). ¹³C NMR (75 MHz, CDCl₃) δ 134.3, 133.9, 133.3, 129.2, 128.4, 127.2, 126.2, 126.1, 125.5, 125.3, 46.5, 33.6, 24.7. HRMS C₁₅H₁₆S [M]⁺; calculated 228.0967, found: 228.0969.

### Example 5

### 2,5,5-trimethyl-2-(4-((2-methylbutyl)thio)phenyl)-1,3-dioxane

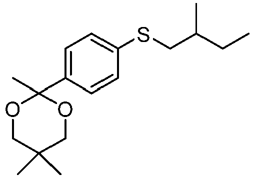

Prepared by general procedure A at 80 °C; isolated as a yellow liquid using pentane/ethyl acetate (30:1) as eluent (109 mg, 71%). ¹H NMR (500 MHz, Chloroform-*d*) δ 7.36-7.27 (m, 4H), 3.45-3.32 (m, 4H), 2.97 (dd, *J* = 12.4, 5.8 Hz, 1H), 2.78 (dd, *J =* 12.4, 7.4 Hz, 1H), 1.79-1.64 (m, 1H), 1.60-1.53 (m, 1H), 1.51 (s, 3H), 1.34-1.27 (m, 1H), 1.26 (s, 3H), 1.04 (d, *J =* 6.7 Hz, 3H), 0.92 (t, *J* = 7.4 Hz, 3H), 0.58 (s, 3H). ¹³C NMR (126 MHz, CDCl₃) δ 138.1, 137.1, 128.4, 127.3, 100.0, 71.7, 40.4, 34.5, 31.9, 29.9, 28.8, 22.9, 21.9, 19.0, 11.3. HRMS C₁₈H₂₈O₂S [M]⁺; calculated 308.1810, found: 308.1809.

### Example 6

### cyclopentyl(4-methoxyphenyl)sulfane

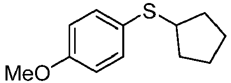

Prepared by general procedure A; isolated as a colorless liquid using pentane/ethyl acetate (50:1) as eluent (95.9 mg, 93%). ¹H NMR (500 MHz, Chloroform-*d*) δ 7.37 (d, *J* = 8.8 Hz, 2H), 6.84 (d, *J* = 8.8 Hz, 2H), 3.80 (s, 3H), 3.48-3.38 (m, 1H), 2.01-1.89 (m, 2H), 1.81-1.73 (m, 2H), 1.63-1.50 (m, 4H). ¹³C NMR (126 MHz, CDCl₃) δ 159.0, 134.1, 127.0, 114.4, 55.3, 48.0, 33.4, 24.6. HRMS C₁₂H₁₆SO [M]⁺; calculated 208.0916, found: 208.0919. The spectral data are consistent with those reported in the literature.

### Example 7

### sec-butyl(4-(methoxymethoxy)phenyl)sulfane

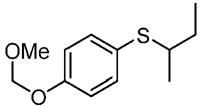

Prepared by general procedure A; isolated as a colorless liquid using pentane/ethyl acetate (30:1) as eluent (110.3 mg, 98%). ¹H NMR (300 MHz, Chloroform-*d*) δ 7.43-7.32 (m, 2H), 7.03-6.87 (m, 2H), 5.16 (s, 2H), 3.48 (s, 3H), 2.99 (td, *J =* 6.9, 6.1 Hz, 1H), 1.62-1.38 (m, 2H), 1.23 (d, *J =* 6.7 Hz, 3H), 0.99 (t, *J* = 7.4 Hz, 3H). ¹³C NMR (75 MHz, Chloroform-*d*) δ 156.8, 135.2, 126.9, 116.6, 94.4, 56.0, 46.1, 29.4, 20.5, 11.5. HRMS C₁₂H₁₈O₂S [M+H]⁺; calculated 227.1100, found: 227.1102.

### Example 8

### triisopropyl(4-((2-methylbutyl)thio)phenoxy)silane

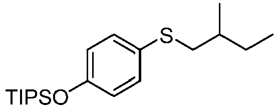

Prepared by general procedure A; isolated as a colorless liquid using pentane/ethyl acetate (50:1) as eluent (136.1 mg, 78%). ¹H NMR (300 MHz, Chloroform-*d*) δ 7.35-7.19 (m, 2H), 6.89-6.76 (m, 2H), 2.87 (dd, *J* = 12.6, 5.7 Hz, 1H), 2.68 (dd, *J* = 12.6, 7.4 Hz, 1H), 1.72-1.45 (m, 2H), 1.26 (dd, *J* = 14.8, 6.8 Hz, 4H), 1.19-1.06 (m, 18H), 1.01 (d, *J* = 6.6 Hz, 3H), 0.89 (t, *J* = 7.4 Hz, 3H). ¹³C NMR (75 MHz, CDCl₃) δ 155.0, 132.3, 128.0, 120.4, 42.8, 34.6, 28.7, 18.8, 17.9, 12.6, 11.2. HRMS C₂₀H₃₆OSSi [M]⁺; calculated 352.2251, found: 352.2249.

### Example 9

### (2-methylbutyl)(4-(trifluoromethoxy)phenyl)sulfane

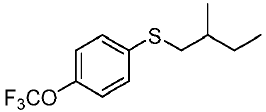

Prepared by general procedure A for 7 h; isolated as a pale yellow liquid using pentane/ethyl acetate (50:1) as eluent (117 mg, 89%). ¹H NMR (300 MHz, Chloroform-d) δ 7.38-7.31 (m, 2H), 7.19-7.11 (m, 2H), 2.96 (dd, *J =* 12.5, 5.8 Hz, 1H), 2.77 (dd, *J =* 12.5, 7.4 Hz, 1H), 1.76-1.48 (m, 2H), 1.37-1.23 (m, 1H), 1.05 (d, *J* = 6.6 Hz, 3H), 0.93 (t, *J =* 7.4 Hz, 3H). ¹⁹F NMR (282 MHz, Chloroform-d) δ - 58.0. ¹³C NMR (75 MHz, Chloroform-d) δ 147.1 (q, *J* = 1.9 Hz), 136.4, 129.9, 121.4, 120.4 (q, *J =* 257.0 Hz), 41.0, 34.4, 28.7, 18.9, 11.2. HRMS C₁₂H₁₅SOF₃ [M]⁺; calculated 264.0790, found: 264.0792.

### Example 10

### (4-(benzyloxy)phenyl)(sec-butyl)sulfane

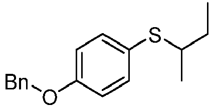

Prepared by general procedure A; isolated as a pale yellow liquid using pentane/ethyl acetate (50:1) as eluent (113.3 mg, 83%). ¹H NMR (300 MHz, Chloroform-*d*) δ 7.48-7.30 (m, 7H), 6.92 (d, *J* = 8.8 Hz, 2H), 5.05 (s, 2H), 2.97 (td, *J =* 6.9, 6.1 Hz, 1H), 1.70-1.39 (m, 2H), 1.22 (d, *J =* 6.8 Hz, 3H), 1.00 (t, *J =* 7.4 Hz, 3H). ¹³C NMR (75 MHz, Chloroform-*d*) δ 158.5, 136.8, 135.5, 128.6, 128.0, 127.5, 125.6, 115.2, 70.1, 46.2, 29.4, 20.5, 11.5. HRMS C₁₇H₂₀OS [M]⁺; calculated 272.1229, found: 272.1232.

### Example 11

### (2-methylbutyl)(4-(trifluoromethyl)phenyl)sulfane

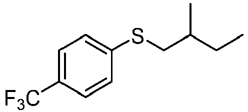

Prepared by general procedure A for 1 h; isolated as a pale yellow liquid using pentane/ethyl acetate (80:1) as eluent (100.1 mg, 81%). ¹H NMR (300 MHz, Chloroform-*d*) δ 7.52 (dt, *J* = 8.3, 0.7 Hz, 2H), 7.41-7.34 (m, 2H), 3.01 (dd, *J =* 12.5, 5.8 Hz, 1H), 2.82 (dd, *J* = 12.5, 7.5 Hz, 1H), 1.79-1.69 (m, 1H), 1.62-1.51 (m, 1H), 1.34 (dd, *J* = 14.3, 6.7 Hz, 1H), 1.07 (d, *J* = 6.6 Hz, 3H), 0.95 (t, *J* = 7.4 Hz, 3H). ¹⁹F NMR (282 MHz, Chloroform-*d*) δ -62.4. ¹³C NMR (126 MHz, Chloroform-*d*) δ 143.2, 127.1, 127.0 (q, *J* = 32.4 Hz), 125.5 (q, *J =* 3.8 Hz), 124.2 (q, *J* = 271.6 Hz), 39.5, 34.4, 28.8, 19.0, 11.2. HRMS C₁₂H₁₅SF₃ [M]⁺; calculated 248.0841, found: 248.0843.

### Example 12

### cyclohexyl(4-fluorophenyl)sulfane

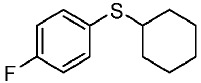

Prepared by general procedure A; isolated as a colorless liquid using pentane/ethyl acetate (100:1) as eluent (78.5 mg, 75%). ¹H NMR (300 MHz, Chloroform-*d*) δ 7.42 (dd, *J* = 8.8, 5.3 Hz, 2H), 7.01 (t, *J* = 8.7 Hz, 2H), 3.00 (ddt, *J* = 10.3, 7.2, 3.8 Hz, 1H), 1.99-1.90 (m, 2H), 1.85-1.71 (m, 2H), 1.66-1.56 (m, 1H), 1.44-1.09 (m, 5H). ¹⁹F NMR (282 MHz, Chloroform-d) δ -114.9. ¹³C NMR (75 MHz, Chloroform-d) δ 162.2 (d, *J =* 246.8 Hz), 134.9 (d, *J* = 8.1 Hz), 129.8 (d, *J =* 3.4 Hz), 115.7 (d, *J* = 21.6 Hz), 47.5, 33.3, 26.0, 25.7. HRMS C₁₂H₁₅FS [M]⁺; calculated 210.0873, found: 210.0872. The spectral data are consistent with those reported in the literature.

### Example 13

### (4-(cyclopentylthio)phenyl)trimethylsilane

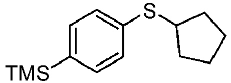

Prepared by general procedure A; isolated as a colorless liquid using pentane as eluent (109.7 mg, 88%). ¹H NMR (300 MHz, Chloroform-d) δ 7.42 (d, *J* = 8.2 Hz, 2H), 7.32 (d, *J* = 8.2 Hz, 2H), 3.62 (ddt, *J* = 7.0, 3.4, 1.6 Hz, 1H), 2.23-2.00 (m, 2H), 1.88-1.72 (m, 2H), 1.72-1.56 (m, 4H), 0.25 (s, 9H). ¹³C NMR (126 MHz, CDCl₃) δ 139.5, 138.4, 134.8, 129.6, 46.4, 34.7, 26.0, 0.0. HRMS C₁₄H₂₂SSi [M]⁺; calculated 250.1206, found: 250.1204.

### Example 14

### 4-(sec-butylthio)-N,N-dimethylaniline

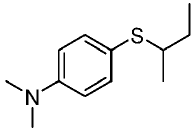

Prepared by general procedure A; isolated as a pale yellow liquid using pentane/ethyl acetate (50:1) as eluent (91.8 mg, 88%). ¹H NMR (300 MHz, Chloroform-*d*) δ 7.41-7.30 (m, 2H), 6.79-6.55 (m, 2H), 2.96 (s, 6H), 2.89 (q, *J =* 6.7 Hz, 1H), 1.69-1.37 (m, 2H), 1.21 (d, *J* = 6.7 Hz, 3H), 0.99 (t, *J* = 7.4 Hz, 3H). ¹³C NMR (126 MHz, Chloroform-*d*) δ 136.0 (2C), 112.6 (2C), 46.4, 40.5, 29.4, 20.5, 11.6. HRMS C₁₂H₁₉NS [M+H]⁺; calculated 210.1311, found: 210.1308.

### Example 15

### 4-(cyclopentylthio)benzonitrile

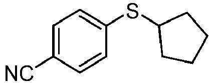

Prepared by general procedure A for 5 h at 80 °C; isolated as a pale yellow liquid using pentane/ethyl acetate (50:1) as eluent (49 mg, 44%). ¹H NMR (300 MHz, Chloroform-*d*) δ 7.49-7.39 (m, 2H), 7.28-7.21 (m, 2H), 3.74-3.53 (m, 1H), 2.08 (dddd, *J* = 9.4, 4.8, 2.5, 1.0 Hz, 2H), 1.73 (d, *J* = 1.4 Hz, 2H), 1.66-1.47 (m, 4H). ¹³C NMR (75 MHz, Chloroform-*d*) δ 145.8, 132.0, 127.2, 118.9, 107.7, 44.0, 33.3, 24.9. HRMS C₁₂H₁₃NS [M+Na]⁺; calculated 226.0661, found: 226.0661.

### Example 16

### 2-(cyclopentylthio)benzo[d]thiazole

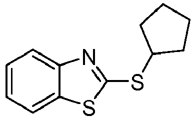

Prepared by general procedure A for 5 h; isolated as a yellow liquid using pentane/ethyl acetate (30:1) as eluent (75.2 mg, 64%). ¹H NMR (500 MHz, Chloroform-*d*) δ 7.88 (dd, *J* = 8.2, 1.0 Hz, 1H), 7.76 (dd, *J* = 7.9, 1.1 Hz, 1H), 7.41 (ddd, *J* = 8.3, 7.2, 1.2 Hz, 1H), 7.32-7.27 (m, 1H), 4.20-4.07 (m, 1H), 2.34-2.23 (m, 2H), 1.90-1.64 (m, 6H). ¹³C NMR (75 MHz, Chloroform-*d*) δ 167.5, 153.4, 135.2, 126.0, 124.1, 121.5, 120.8, 46.7, 33.8, 24.9. HRMS C₁₂H₁₃NS₂ [M+Na]⁺; calculated 258.0382, found: 258.0379. The spectral data are consistent with those reported in the literature.

### Example 17

### 2-(cyclohexylthio)pyridine

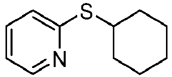

Prepared by general procedure A for 2 h at 70 °C; isolated as a pale yellow liquid using pentane/ethyl acetate (20:1) as eluent (83 mg, 86%). ¹H NMR (300 MHz, Chloroform-*d*) δ 8.42 (dt, *J* = 4.8, 1.5 Hz, 1H), 7.45 (ddd, *J* = 8.0, 7.3, 1.9 Hz, 1H), 7.18-7.12 (m, 1H), 6.95 (ddd, *J =* 7.4, 4.9, 1.1 Hz, 1H), 3.82 (ddd, *J =* 10.1, 6.2, 3.7 Hz, 1H), 2.13-2.01 (m, 2H), 1.85-1.69 (m, 2H), 1.69-1.59 (m, 1H), 1.54-1.22 (m, 5H). ¹³C NMR (75 MHz, Chloroform-d) δ 159.2, 149.3, 136.0, 123.0, 119.3, 43.0, 33.3, 26.0, 25.8. HRMS C₁₁H₁₅NS [M+H]⁺; calculated 194.0997, found: 194.0998. The spectral data are consistent with those reported in the literature.

### Example 18

### 2-(cyclohexylthio)pyrazine

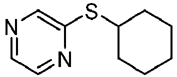

Prepared by general procedure A for 2 h at 80 °C; isolated as a pale yellow liquid using pentane/ethyl acetate (50:1) as eluent (77.6 mg, 80%). ¹H NMR (300 MHz, Chloroform-d) δ 8.42 (d, *J* = 1.6 Hz, 1H), 8.37 (dd, *J* = 2.7, 1.6 Hz, 1H), 8.19 (d, *J* = 2.7 Hz, 1H), 3.93-3.78 (m, 1H), 2.18-2.04 (m, 2H), 1.87-1.73 (m, 2H), 1.67 (ddt, *J* = 9.6, 6.3, 2.4 Hz, 1H), 1.58-1.29 (m, 5H). ¹³C NMR (75 MHz, Chloroform-*d*) δ 157.3, 144.2, 143.9, 139.2, 42.9, 33.1, 25.9, 25.7. HRMS C₁₀H₁₄N₂S [M+H]⁺; calculated 195.0950, found: 195.0950.

### Example 19

### dodecyl(4-methoxyphenyl)sulfane

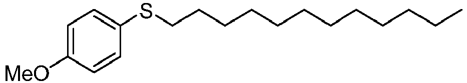

Prepared by general procedure A; isolated as a pale yellow liquid using pentane/ethyl acetate (50:1) as eluent (135 mg, 88%). ¹H NMR (300 MHz, Chloroform-*d*) δ 7.35 (d, *J* = 8.8 Hz, 2H), 6.86 (d, *J* = 8.8 Hz, 2H), 3.82 (s, 3H), 2.84 (br, 2H), 1.72-1.50 (m, 2H), 1.43-1.28 (m, 18H), 0.98-0.82 (m, 3H). ¹³C NMR (126 MHz, CDCl₃) δ 158.7, 132.9, 114.8, 114.5, 55.3, 35.8, 31.9, 29.7, 29.63, 29.59, 29.5, 29.40, 29.35, 29.2, 28.7, 22.7, 14.1. HRMS C₁₉H₃₂OS [M]⁺; calculated 308.2168, found: 308.2167. The spectral data are consistent with those reported in the literature.

### Example 20

### ((3s,5s,7s)-adamantan-1-yl)(phenyl)sulfane

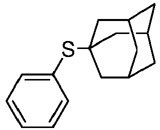

Prepared by general procedure A; isolated as a white solid using pentane as eluent (95 mg, 78%). ¹H NMR (500 MHz, Chloroform-*d*) δ 7.56-7.48 (m, 2H), 7.39-7.29 (m, 3H), 2.06-1.95 (m, 3H), 1.82 (d, *J* = 2.9 Hz, 6H), 1.70-1.56 (m, 6H). ¹³C NMR (126 MHz, CDCl₃) δ 137.7, 130.5, 128.6, 128.3, 47.8, 43.6, 36.2, 30.0. HRMS C₁₆H₂₀S [M]⁺; calculated 244.1280, found: 244.1280.

### Example 21

### phenethyl(phenyl)sulfane

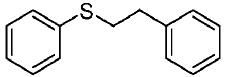

Prepared by general procedure A; isolated as a colorless liquid using pentane/ethyl acetate (100:1) as eluent (60.5 mg, 57%). ¹H NMR (500 MHz, Chloroform-*d*) δ 7.21-7.15 (m, 2H), 7.11 (td, *J =* 7.7, 3.1 Hz, 4H), 7.07-6.96 (m, 4H), 3.04-2.93 (m, 2H), 2.80-2.69 (m, 2H). ¹³C NMR (126 MHz, CDCl₃) δ 140.2, 136.4, 129.2, 128.9, 128.6, 128.5, 126.5, 126.0, 35.7, 35.1. HRMS C₁₄H₁₄S [M]⁺; calculated 214.0816, found: 214.0816. The spectral data are consistent with those reported in the literature.

### Example 22

### phenyl((1S,2S,5R)-2,6,6-trimethylbicyclo[3.1.1]heptan-2-yl)sulfane

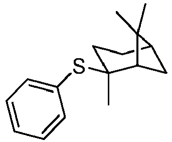

Prepared by general procedure A; isolated as a colorless liquid using pentane/ethyl acetate (100:1) as eluent (114 mg, 93%). ¹H NMR (500 MHz, Chloroform-*d*) δ 7.41-7.32 (m, 2H), 7.32-7.27 (m, 2H), 7.24-7.13 (m, 1H), 3.19-2.79 (m, 2H), 2.46-2.24 (m, 2H), 2.14-1.82 (m, 5H), 1.74-1.53 (m, 1H), 1.22 (s, 3H), 1.06 (s, 3H), 0.91 (d, *J* = 9.7 Hz, 1H). ¹³C NMR (126 MHz, CDCl₃) δ 137.3, 128.9, 128.8, 125.6, 45.6, 41.3, 40.8, 40.6, 38.7, 33.3, 28.0, 26.2, 23.3, 22.1. HRMS C₁₆H₂₂S [M]⁺; calculated 246.1436, found: 246.1437.

### Example 23

### octyl(phenyl)sulfane

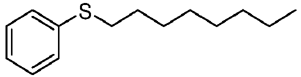

Prepared by general procedure A; isolated as a colorless liquid using pentane as eluent (102 mg, 92%). ¹H NMR (500 MHz, Chloroform-*d*) δ 7.15 (dd, *J* = 8.6, 1.4 Hz, 2H), 7.09 (dd, *J* = 8.6, 6.9 Hz, 2H), 7.01-6.94 (m, 1H), 2.82-2.69 (m, 2H), 1.63-1.40 (m, 2H), 1.33-1.20 (m, 2H), 1.16-1.03 (m, 8H), 0.71 (t, *J* = 7.0 Hz, 3H). ¹³C NMR (126 MHz, CDCl₃) δ 137.1, 128.84, 128.81, 125.6, 33.6, 31.9, 29.23, 29.20, 29.19, 28.9, 22.7, 14.1. HRMS C₁₄H₂₂S [M]⁺; calculated 222.1442, found: 222.1441. The spectral data are consistent with those reported in the literature.

### Example 24

### benzyl(phenyl)sulfane

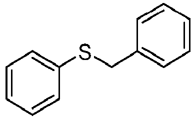

Prepared by general procedure A for 1h; isolated as a colorless liquid using pentane as eluent (76.6 mg, 77%). ¹H NMR (500 MHz, Chloroform-*d*) δ 7.37-7.26 (m, 9H), 7.25-7.19 (m, 1H), 4.16 (s, 2H). ¹³C NMR (126 MHz, CDCl₃) δ 137.5, 136.4, 129.8, 128.9, 128.8, 128.5, 127.2, 126.4, 39.1. HRMS C₁₃H₁₂S [M]⁺; calculated 200.0654, found: 200.0654. The spectral data are consistent with those reported in the literature (45).

### Example 25

### 1,3-bis(cyclohexylthio)benzene

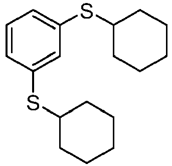

Prepared by general procedure A (0.2 mmol scale); isolated as a pale yellow liquid using pentane/ethyl acetate (100:1) as eluent (53.9 mg, 88%). ¹H NMR (500 MHz, Chloroform-d) δ 7.41 (s, 1H), 7.24-7.15 (m, 3H), 3.30-3.00 (m, 2H), 2.07-1.91 (m, 4H), 1.84-1.74 (m, 4H), 1.69-1.59 (m, 2H), 1.44-1.17 (m, 10H). ¹³C NMR (126 MHz, CDCl₃) δ 135.9, 134.2, 129.7, 128.9, 46.5, 33.3, 26.0, 25.8. HRMS C₁₈H₂₆S₂ [M]⁺; calculated 306.1476, found: 306.1478.

### Example 26

### ((3S,10R,13R)-10,13-dimethyl-17-((R)-6-methylheptan-2-yl)-2,3,4,7,8,9,10,11,12,13,14,15,16,17-tetradecahydro-1H-cyclopenta[a]phenanthren-3-yl)(phenyl)sulfane

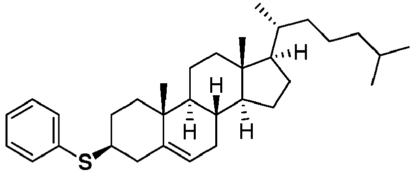

Prepared by general procedure A (0.2 mmol scale); isolated as a white solid using pentane as eluent (66.6mg, 70%). ¹H NMR (500 MHz, Chloroform-*d*) δ 7.42 (dd, *J* = 8.2, 1.3 Hz, 2H), 7.31 (dd, *J* = 8.2, 6.8 Hz, 2H), 7.28-7.20 (m, 1H), 5.34 (dd, *J =* 5.1, 1.7 Hz, 1H), 3.17-2.95 (m, 1H), 2.35 (d, *J =* 8.2 Hz, 2H), 2.08-1.95 (m, 2H), 1.92 (dt, *J =* 9.5, 3.7 Hz, 2H), 1.90-1.80 (m, 1H), 1.69-1.44 (m, 12H), 1.45-1.05 (m, 9H), 1.02 (s, 3H), 0.95 (d, *J* = 6.5 Hz, 3H), 0.91 (d, *J* = 2.2 Hz, 3H), 0.89 (d, *J =* 2.2 Hz, 3H), 0.70 (s, 3H). ¹³C NMR (126 MHz, CDCl₃) δ 141.7, 134.9, 131.8, 128.8, 126.6, 121.2, 56.8, 56.2, 50.3, 47.4, 42.3, 39.8, 39.7, 39.5, 36.9, 36.2, 35.8, 31.9, 31.8, 29.5, 28.2, 28.0, 24.3, 23.9, 22.8, 22.6, 20.9, 19.4, 18.7, 11.9. [α]_{D}²⁵= - 37.7 (c= 0.52, CH₂Cl₂). HRMS C₃₃H₅₀S [M]⁺; calculated 478.3633, found: 478.3634.

### Example 27

### cyclohexyl(phenyl)sulfane

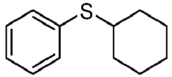

Prepared by general procedure A; isolated as a colorless liquid using pentane as eluent (86.2 mg, 90%). ¹H NMR (500 MHz, Chloroform-*d*) δ 7.41-7.38 (m, 2H), 7.30-7.26 (m, 2H), 7.23-7.18 (m, 1H), 3.17-3.01 (m, 1H), 2.02-1.95 (m, 2H), 1.82-1.73 (m, 2H), 1.66-1.58 (m, 1H), 1.44-1.24 (m, 5H). ¹³C NMR (125 MHz, CDCl₃) δ 135.2, 131.9, 128.7, 126.6, 46.6, 33.4, 26.1, 25.8. HRMS C₁₂H₁₆S [M]⁺; calculated 192.0967, found: 192.0969. The spectral data are consistent with those reported in the literature.

### Example 28

### cyclohexyl(styryl)sulfane

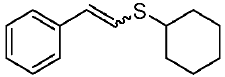

Prepared from the corresponding starting material (*E*/*Z* 5:1) by general procedure A at 160 °C; isolated as a pale yellow liquid using pentane/ethyl acetate (100:1) as eluent (89 mg, 82%, *E*/*Z =* 5:1). E isomer: ¹H NMR (500 MHz, Chloroform-*d*) δ 7.35-7.30 (m, 4H), 7.25-7.19 (m, 1H), 6.80 (d, *J =* 15.6 Hz, 1H), 6.61 (d, *J =* 15.6 Hz, 1H), 3.05-3.01 (m, 1H), 2.20-2.03 (m, 2H), 1.92-1.78 (m, 2H), 1.73-1.64 (m, 1H), 1.55-1.07 (m, 6H). ¹³C NMR (126 MHz, CDCl₃) δ 137.2, 128.6, 128.6, 126.9, 125.6, 124.1, 45.3, 33.6, 26.0, 25.7. Z isomer: ¹H NMR (500 MHz, Chloroform-*d*) δ 7.58-7.49 (m, 2H), 7.38 (t, *J* = 7.8 Hz, 2H), 7.24 (s, 1H), 6.47 (d, *J* = 11.0 Hz, 1H), 6.37 (d, *J* = 11.0 Hz, 0H), 2.97-2.88 (m, 1H), 2.09-2.07 (m, 2H), 1.85-1.82 (m, 2H), 1.69-1.66 (m, 1H), 1.58-1.28 (m, 6H). ¹³C NMR (126 MHz, CDCl₃, characteristic peak) 137.2, 128.2, 126.5, 125.9, 125.0, 47.8, 33.7, 25.6. HRMS C₁₄H₁₈S [M]⁺; calculated 218.1129, found: 218.1129. The spectral data are consistent with those reported in the literature.

### General procedure for the catalytic aryl transfer with thiophenols

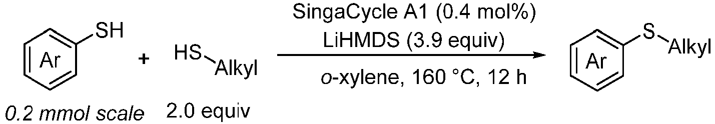

In the glovebox, aryl thiol (0.2 mmol), alkyl thiol (2.0 equiv, 0.4 mmol), LiHMDS (3.9 equiv, 135 mg), and SingaCycle A1 (0.4 mol%, 0.2 ml, 0.005 M in *o*-xylene) were added into the oven-dried 8 ml vial with a magnetic stirring bar, followed by addition of *o*-xylene (1.8 ml). The vial was sealed and removed out of the glovebox and heated to 160 °C. After 12 h, the vial was cooled to room temperature. The reaction was diluted with ethyl acetate and washed by saturated NaOH solution. The aqueous phase was extracted with ethyl acetate 3 times. The collected organic phases were dried over anhydrous Na₂SO₄. The solvent was removed under reduced pressure and the residue was purified by flash column chromatography to give the desired product.

### Example 29

### (4-methoxyphenyl)(octyl)sulfane

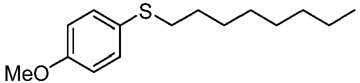

Prepared by general procedure B; isolated as a pale yellow liquid using pentane/ethyl acetate (50:1) as eluent (38 mg, 76%). ¹H NMR (300 MHz, Chloroform-*d*) δ 7.34 (d, *J* = 8.9 Hz, 2H), 6.84 (d, *J* = 8.8 Hz, 2H), 3.79 (s, 3H), 2.99-2.71 (m, 2H), 1.65-1.50 (m, 2H), 1.45-1.17 (m, 10H), 0.97-0.75 (m, 3H). ¹³C NMR (75 MHz, CDCl₃) δ 158.7, 132.8, 127.0, 114.4, 55.3, 35.8, 31.8, 29.3, 29.1, 29.0, 28.7, 22.6, 14.1. HRMS C₁₅H₂₄SO [M]⁺; calculated 252.1542, found: 252.1544. The spectral data are consistent with those reported in the literature.

### Example 30

### cyclohexyl(p-tolyl)sulfane

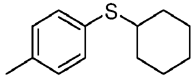

Prepared by general procedure B; isolated as a colorless liquid using pentane/ethyl acetate (100:1) as eluent (30.5 mg, 75%). See above for experimental data.

### Example 31

### cyclopentyl(o-tolyl)sulfane

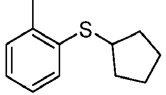

Prepared by general procedure B; isolated as a colorless liquid using pentane/ethyl acetate (100:1) as eluent (29.3 mg, 77%). ¹H NMR (500 MHz, Chloroform-d) δ 7.39-7.29 (m, 1H), 7.15 (t, *J* = 7.7 Hz, 2H), 7.11-7.06 (m, 1H), 3.71-3.49 (m, 1H), 2.37 (s, 3H), 2.17-2.03 (m, 2H), 1.84-1.75 (m, 2H), 1.72-1.56 (m, 4H). ¹³C NMR (126 MHz, CDCl₃) δ 137.5, 136.8, 130.0, 128.8, 126.2, 125.4, 44.9, 33.6, 24.9, 20.5. HRMS C₁₂H₁₆S [M]⁺; calculated 192.0973, found: 192.0970. The spectral data are consistent with those reported in the literature.

### Example 32

### cyclohexyl(phenyl)sulfane

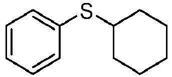

Prepared by general procedure B; isolated as a colorless liquid using pentane as eluent (32.3 mg, 85%). See above for experimental data.

### Example 33

### (4-(tert-butyl)phenyl)(2-methylbutyl)sulfane

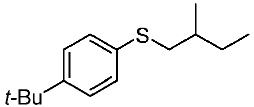

Prepared by general procedure B; isolated as a colorless liquid using pentane/ethyl acetate (100:1) as eluent (42.6 mg, 91%). ¹H NMR (500 MHz, Chloroform-d) δ 7.44-7.27 (m, 4H), 2.97 (dd, *J =* 12.5, 5.9 Hz, 1H), 2.77 (dd, *J =* 12.5, 7.5 Hz, 1H), 1.87-1.66 (m, 1H), 1.63-1.51 (m, 1H), 1.34 (s, 9H), 1.32-1.24 (m, 1H), 1.06 (d, *J* = 6.7 Hz, 3H), 0.94 (t, *J* = 7.4 Hz, 3H). ¹³C NMR (126 MHz, CDCl₃) δ 148.8, 133.9, 128.9, 125.8, 41.2, 34.6, 34.4, 31.3, 28.8, 18.9, 11.3. HRMS C₁₅H₂₄S [M]⁺; calculated 236.1599, found: 236.1599.

### Example 34

### 4-(cyclohexylthio)pyridine

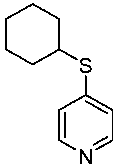

Prepared by general procedure B; isolated as a colorless liquid using pentane/ethyl acetate (20:1) as eluent (18 mg, 47%). ¹H NMR (500 MHz, Chloroform-d) δ 8.37 (d, *J =* 6.3 Hz, 2H), 7.11 (d, *J =* 6.3 Hz, 2H), 3.48-3.25 (m, 1H), 2.10-1.95 (m, 2H), 1.86-1.74 (m, 2H), 1.68-1.64 (m, 1H), 1.54-1.35 (m, 5H). ¹³C NMR (126 MHz, CDCl₃) δ 149.1, 148.8, 121.7, 43.4, 32.9, 25.8, 25.6. HRMS C₁₁H₁₅NS [M+H]⁺; calculated 194.0997, found: 194.0997. The spectral data are consistent with those reported in the literature.

### Example 35

### (3,5-dimethylphenyl)(2-methylbutyl)sulfane

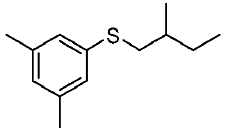

Prepared by general procedure B; isolated as a colorless liquid using pentane/ethyl acetate (100:1) as eluent (37.6 mg, 91%). ¹H NMR (500 MHz, Chloroform-d) δ 6.98 (s, 2H), 6.82 (s, 1H), 2.97 (dd, *J =* 12.4, 5.8 Hz, 1H), 2.77 (dd, *J =* 12.4, 7.5 Hz, 1H), 2.32 (s, 6H), 1.78-1.65 (m, 1H), 1.63-1.54 (m, 1H), 1.31 (dt, *J* = 13.5, 7.5 Hz, 1H), 1.06 (d, *J* = 6.6 Hz, 3H), 0.95 (t, *J =* 7.5 Hz, 3H). ¹³C NMR (126 MHz, CDCl₃) δ 138.4, 137.1, 127.4, 126.4, 40.6, 34.6, 28.8, 21.3, 19.0, 11.3. HRMS C₁₃H₂₀S [M]⁺; calculated 208.1286, found: 208.1284. The spectral data are consistent with those reported in the literature.

### Example 36

### (4-methoxyphenyl)(2-methylbutyl)sulfane

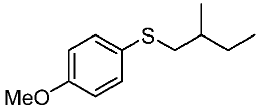

Prepared by general procedure B; isolated as a pale yellow liquid using pentane/ethyl acetate (40:1) as eluent (33.9 mg, 81%). ¹H NMR (300 MHz, Chloroform-d) δ 7.41-7.27 (m, 2H), 6.91-6.78 (m, 2H), 3.79 (s, 3H), 2.85 (dd, *J =* 12.6, 5.7 Hz, 1H), 2.66 (dd, *J* = 12.6, 7.4 Hz, 1H), 1.68-1.45 (m, 2H), 1.35-1.17 (m, 1H), 0.99 (d, *J* = 6.6 Hz, 3H), 0.87 (t, *J* = 7.4 Hz, 3H). ¹³C NMR (75 MHz, CDCl₃) δ 158.6, 132.6, 127.6, 114.5, 55.3, 43.0, 34.5, 28.6, 18.8, 11.2. HRMS C₁₂H₁₈SO [M]⁺; calculated 210.1078, found: 210.1079.

### Example 37

### 2-((2-methylbutyl)thio)-5-phenylbenzo[d]thiazole

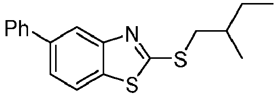

Prepared by general procedure B; isolated as a pink solid using pentane/ethyl acetate (50:1) as eluent (23 mg, 37%). ¹H NMR (300 MHz, Chloroform-d) δ 8.08 (s, 1H), 7.80 (d, *J* = 8.3 Hz, 1H), 7.66 (d, *J* = 7.5 Hz, 2H), 7.55-7.37 (m, 4H), 3.44 (dd, *J* = 12.7, 5.9 Hz, 1H), 3.24 (dd, *J* = 12.7, 7.4 Hz, 1H), 1.97-1.76 (m, 1H), 1.71-1.52 (m, 1H), 1.39-1.30 (m, 1H), 1.08 (d, *J =* 6.7 Hz, 3H), 0.97 (t, *J* = 7.4 Hz, 3H). ¹³C NMR (126 MHz, CDCl₃) δ 168.5, 154.0, 140.8, 139.6, 134.1, 128.9, 127.4, 127.3, 123.5, 121.0, 119.8, 40.4, 34.9, 28.7, 18.9, 11.3. HRMS C₁₈H₁₉S₂N [M]⁺; calculated 314.1032, found: 314.1033.

### Example 38

### cyclohexyl(naphthalen-1-yl)sulfane

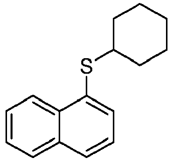

Prepared by general procedure B; isolated as a colorless liquid using pentane/ethyl acetate (100:1) as eluent (33.5 mg, 70%). ¹H NMR (300 MHz, Chloroform-d) δ 8.63-8.49 (m, 1H), 7.89-7.75 (m, 2H), 7.71 (dd, *J =* 7.2, 1.2 Hz, 1H), 7.55 (ddd, *J* = 10.5, 7.9, 1.4 Hz, 2H), 7.42 (dd, *J* = 8.2, 7.2 Hz, 1H), 3.31-3.04 (m, 1H), 2.06-1.92 (m, 2H), 1.82-1.73 (m, 2H), 1.65-1.57 (m, 1H), 1.52-1.12 (m, 5H). ¹³C NMR (75 MHz, CDCl₃) δ 134.4, 134.0, 132.3, 131.8, 128.4, 128.1, 126.3, 126.0, 125.9, 125.4, 47.2, 33.5, 26.0, 25.8. HRMS C₁₆H₁₈S [M]⁺; calculated 242.1124, found: 242.1123.

### Example 39

### cyclohexyl(naphthalen-2-yl)sulfane

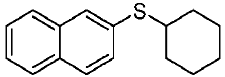

Prepared by general procedure B; isolated as a colorless liquid using pentane/ethyl acetate (100:1) as eluent (40 mg, 83%). ¹H NMR (300 MHz, Chloroform-d) δ 7.87 (d, *J* = 1.8 Hz, 1H), 7.79 (ddd, *J* = 12.7, 7.9, 2.4 Hz, 3H), 7.56-7.41 (m, 3H), 3.37-3.17 (m, 1H), 2.19-1.99 (m, 2H), 1.85-1.73 (m, 2H), 1.71-1.59 (m, 1H), 1.52-1.20 (m, 5H). ¹³C NMR (75 MHz, CDCl₃) δ 133.6, 132.6, 132.0, 130.1, 129.6, 128.2, 127.6, 127.2, 126.3, 125.8, 46.5, 33.3, 26.0, 25.8. HRMS C₁₆H₁₈S [M]⁺; calculated 242.1124, found: 242.1123.

### Example 40

### ((3s,5s,7s)-adamantan-1-yl)(phenyl)sulfane

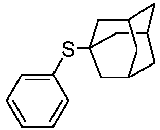

Prepared by general procedure B; isolated as a white solid using pentane as eluent (34.1 mg, 70%). See above for experimental data.

### Example 41

### phenyl((1S,2S,5R)-2,6,6-trimethylbicyclo[3.1.1]heptan-2-yl)sulfane

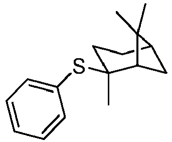

Prepared by general procedure B; isolated as a colorless liquid using pentane/ethyl acetate (100:1) as eluent (45 mg, 92%). See above for experimental data.

### Example 42

### N,N-dimethyl-2-(phenylthio)ethan-1-amine

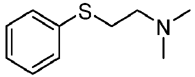

Prepared by general procedure B; isolated as a pale yellow liquid using acetone as eluent (21.6 mg, 60%). ¹H NMR (300 MHz, Chloroform-d) δ 7.41-7.34 (m, 2H), 7.33-7.26 (m, 2H), 7.23-7.15 (m, 1H), 3.20-2.93 (m, 2H), 2.83-2.52 (m, 2H), 2.30 (s, 6H). ¹³C NMR (75 MHz, CDCl₃) δ 136.4, 128.9, 128.8, 125.8, 58.5, 45.3, 31.4. HRMS C₁₀H₁₅SN [M+H]⁺; calculated 182.0998, found: 182.0999.

### Example 43

### (2-((3r,5r,7r)-adamantan-1-yl)ethyl)(phenyl)sulfane

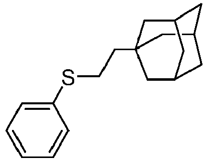

Prepared by general procedure B; isolated as a colorless liquid using pentane/ethyl acetate (100:1) as eluent (39.3 mg, 73%). ¹H NMR (500 MHz, Chloroform-*d*) δ 7.39-7.27 (m, 4H), 7.24-7.14 (m, 1H), 3.05-2.76 (m, 2H), 1.99 (q, *J* = 3.0 Hz, 3H), 1.82-1.71 (m, 3H), 1.70-1.63 (m, 3H), 1.55 (d, *J* = 3.0 Hz, 6H), 1.52-1.37 (m, 2H). ¹³C NMR (126 MHz, CDCl₃) δ 137.2, 128.8, 128.4, 125.5, 43.6, 42.2, 37.1, 32.8, 28.6, 27.5. HRMS C₁₈H₂₄S [M]⁺; calculated 272.1599, found: 272.1597.

### Example 44

### phenethyl(phenyl)sulfane

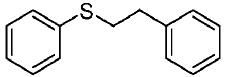

Prepared by general procedure B; isolated as a colorless liquid using pentane/ethyl acetate (100:1) as eluent (30.2 mg, 71%). See above for experimental data.

### Example 45

### diphenylsulfane

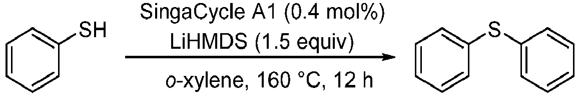

In the glovebox, phenyl thiol (0.2 mmol), LiHMDS (1.5 equiv, 51.6 mg), and SingaCycle A1 (0.4 mol%) were added into an oven-dried 8 ml vial with a magnetic stirring bar, followed by addition of o-xylene (2 ml). The vial was sealed and removed out of the glovebox and heated to 160 °C. After 12 h, the vial was cooled to room temperature. The reaction was diluted with ethyl acetate and washed by saturated NaOH solution. The aqueous phase was extracted with ethyl acetate 3 times. The collected organic phases were dried over anhydrous Na₂SO₄. The solvent was removed under reduced pressure and the residue was purified by flash column chromatography (pentane) to give the title product (14.1 mg, 76%). ¹H NMR (500 MHz, Chloroform-*d*) δ 7.41-7.36 (m, 4H), 7.36-7.31 (m, 4H), 7.30-7.25 (m, 2H). ¹³C NMR (126 MHz, CDCl₃) δ 135.8, 131.1, 129.2, 127.0. HRMS C₁₂H₁₀S [M]⁺; calculated 186.0497, found: 186.0498. The spectral data are consistent with those reported in the literature.

### Example 46

### Diphenylselane

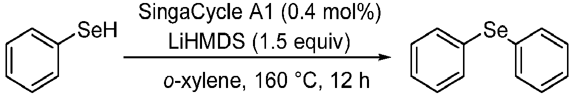

In the glovebox, phenyl selenol (0.2 mmol), LiHMDS (1.5 equiv, 51.6 mg), and SingaCycle A1 (0.4 mol%) were added into an oven-dried 8 ml vial with a magnetic stirring bar, followed by addition of o-xylene (2 ml). The vial was sealed and removed out of the glovebox and heated to 160 °C. After 12 h, the vial was cooled to room temperature. The reaction was diluted with ethyl acetate and washed by saturated NaOH solution. The aqueous phase was extracted with ethyl acetate 3 times. The collected organic phases were dried over anhydrous Na₂SO₄. The solvent was removed under reduced pressure and the residue was purified by flash column chromatography (pentane) to give the title product (22.3 mg, 96%). ¹H NMR (500 MHz, Chloroform-d) δ 7.53 (dq, *J =* 7.7, 3.5 Hz, 4H), 7.31 (dq, *J =* 5.7, 3.0 Hz, 6H). ¹³C NMR (126 MHz, Chloroform-d) δ 133.1 (d, *J =* 3.0 Hz), 131.2 (d, *J* = 4.3 Hz), 129.4 (d, *J =* 3.8 Hz), 127.4 (d, *J* = 2.7 Hz). HRMS C₁₂H₁₀Se [M]⁺; calculated 233.9947, found: 233.9948. The spectral data are consistent with those reported in the literature.

### Procedure for late-stage derivatization

### Example 47

### 2-(cyclohexylthio)-10-(2-(1-methylpiperidin-2-yl)ethyl)-10H-phenothiazine

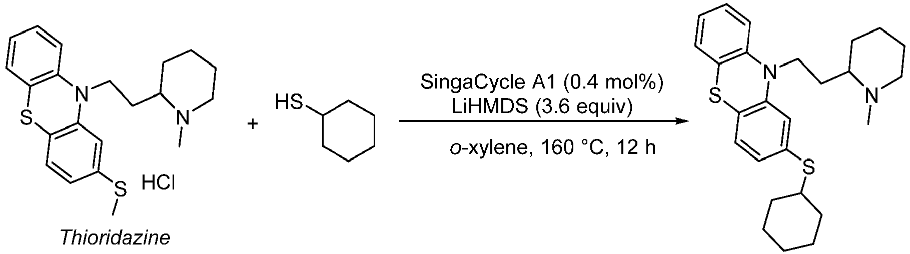

In the glovebox, thioridazine (0.2 mmol), cyclohexyl thiol (2.0 equiv, 0.4 mmol), LiHMDS (3.6 equiv), and SingaCycle A1 (0.4 mol%) were added into an oven-dried 8 ml vial with a magnetic stirring bar, followed by addition of o-xylene (1.0 ml). The vial was sealed and removed out of the glovebox and heated to 160 °C. After 12 h, the vial was cooled to room temperature. The reaction was diluted with ethyl acetate and washed with NaOH solution. The aqueous phase was extracted with ethyl acetate 3 times. The collected organic phases were dried over anhydrous Na₂SO₄. The solvent was removed under reduced pressure and the residue was purified by flash column chromatography (0-5% MeOH in DCM) to give the title product. Isolated as syrup like liquid (54.3 mg, 62%). ¹H NMR (500 MHz, Chloroform-d) δ 7.21-7.11 (m, 2H), 7.05 (d, *J* = 7.9 Hz, 1H), 6.98 (dd, *J =* 7.9, 1.7 Hz, 1H), 6.93 (dd, *J =* 11.5, 1.7 Hz, 2H), 6.89 (dd, *J =* 7.9, 1.1 Hz, 1H), 4.09-3.92 (m, 1H), 3.86 (dt, *J* = 14.2, 7.4 Hz, 1H), 3.14-2.99 (m, 1H), 2.97-2.86 (m, 1H), 2.27-2.23 (m, 6H), 2.04-1.88 (m, 3H), 1.83-1.55 (m, 7H), 1.49-1.12 (m, 7H). ¹³C NMR (126 MHz, CDCl₃) δ 145.4, 144.9, 134.1, 127.6, 127.5, 127.4, 126.5, 125.4, 124.5, 122.8, 119.7, 115.9, 62.3, 56.7, 47.2, 43.8, 42.4, 33.3, 30.2, 29.3, 26.0, 25.7, 24.9, 23.6. HRMS C₂₆H₃₄S₂N₂ [M+H]⁺; calculated 439.2236, found: 439.2239.

### Example 48

### 10-(2-(1-methylpiperidin-2-yl)ethyl)-2-(octylthio)-10H-phenothiazine

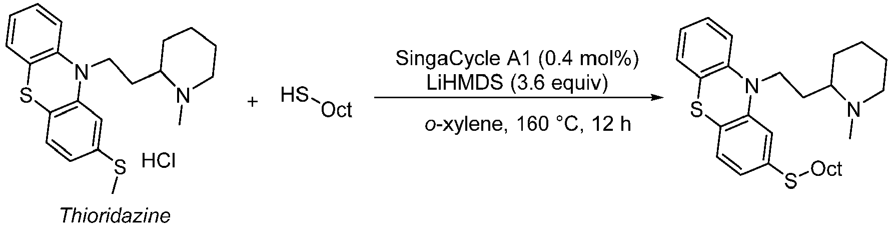

In the glovebox, thioridazine (0.2 mmol), 1-octanethiol (2.0 equiv, 0.4 mmol), LiHMDS (3.6 equiv), and SingaCycle A1 (0.4 mol%) were added into an oven-dried 8 ml vial with a magnetic stirring bar, followed by addition of o-xylene (1.0 ml). Then the vial was sealed and removed out of the glovebox and heated to 160 °C. After 12 h, the vial was cooled to room temperature. The reaction was diluted with ethyl acetate and washed with NaOH solution. The aqueous phase was extracted with ethyl acetate 3 times. The collected organic phases were dried over anhydrous Na₂SO₄. The solvent was removed under reduced pressure and the residue was purified by flash column chromatography (0-5% MeOH in DCM) to give the title product. Isolated as orange oil (46 mg, 49%). ¹H NMR (500 MHz, Chloroform-*d*) δ 7.20-7.09 (m, 2H), 7.04 (d, *J* = 7.9 Hz, 1H), 6.97-6.81 (m, 4H), 3.96 (ddd, *J* = 13.8, 8.5, 5.3 Hz, 1H), 3.84 (ddd, *J =* 13.8, 8.7, 6.1 Hz, 1H), 2.93-2.81 (m, 3H), 2.24 (s, 3H), 2.15 (qt, *J =* 9.8, 7.2, 3.3 Hz, 3H), 1.89 (dtd, *J =* 13.8, 8.0, 4.8 Hz, 1H), 1.77-1.69 (m, 2H), 1.66-1.57 (m, 4H), 1.55-1.43 (m, 1H), 1.43-1.35 (m, 2H), 1.35-1.19 (m, 9H), 0.87 (t, *J* = 6.9 Hz, 3H). ¹³C NMR (126 MHz, CDCl₃) δ 145.8, 145.1, 136.2, 127.7, 127.6, 127.4, 125.5, 123.4, 123.2, 122.8, 117.0, 115.9, 62.4, 57.0, 44.0, 43.0, 34.4, 31.9, 30.7, 29.8, 29.3, 29.3, 29.3, 29.0, 25.5, 24.1, 22.8, 14.2. HRMS C₂₈H₄₀S₂N₂ [M+H]⁺; calculated 469.2705, found: 469.2709.

### Example 49

### 2-(benzylthio)-10-(2-(1-methylpiperidin-2-yl)ethyl)-10H-phenothiazine

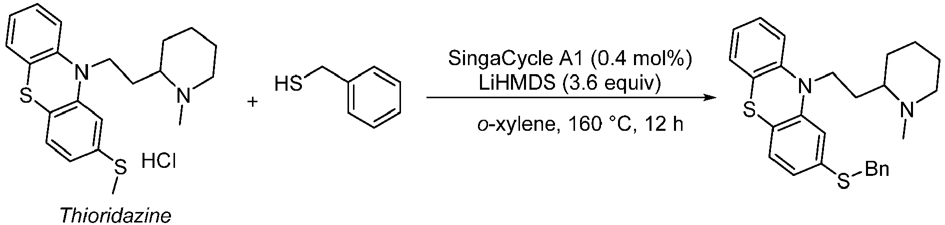

In the glovebox, thioridazine (0.2 mmol), benzyl thiol (2.0 equiv, 0.4 mmol), LiHMDS (3.6 equiv), and SingaCycle A1 (0.4 mol%) were added into an oven-dried 8 ml vial with a magnetic stirring bar, followed by addition of o-xylene (1.0 ml). The vial was sealed and removed out of the glovebox and heated to 160 °C. After 12 h, the vial was cooled to room temperature. The reaction was diluted with ethyl acetate and washed with NaOH solution. The aqueous phase was extracted with ethyl acetate 3 times. The collected organic phases were dried over anhydrous Na₂SO₄. The solvent was removed under reduced pressure and the residue was purified by flash column chromatography (0-5% MeOH in DCM) to give the title product. Isolated as orange oil (49.7 mg, 56%). ¹H NMR (500 MHz, Chloroform-d) δ 7.22-7.12 (m, 5H), 7.10-7.01 (m, 2H), 6.93 (d, *J =* 8.0 Hz, 1H), 6.87-6.77 (m, 2H), 6.76 (dd, *J* = 8.0, 1.2 Hz, 1H), 6.65 (d, *J* = 1.8 Hz, 1H), 3.98 (s, 2H), 3.79 (ddd, *J* = 13.1, 7.7, 5.0 Hz, 1H), 3.64 (dt, *J* = 14.3, 7.7 Hz, 1H), 2.89 (d, *J* = 11.8 Hz, 1H), 2.22-2.06 (m, 6H), 1.86-1.47 (m, 6H), 1.32-1.11 (m, 1H). ¹³C NMR (126 MHz, CDCl₃) δ 145.5, 144.6, 137.5, 135.4, 128.81, 128.80, 128.6, 127.6, 127.5, 127.3, 125.6, 124.4, 124.1, 122.9, 117.7, 116.0, 62.4, 56.5, 43.6, 42.0, 39.4, 29.7, 28.7, 24.4, 23.3. HRMS C₂₇H₃₀S₂N₂ [M+H]⁺; calculated 447.1923, found: 447.1925.

### Example 50

### 2-(sec-butylthio)-10-(2-(1-methylpiperidin-2-yl)ethyl)-10H-phenothiazine

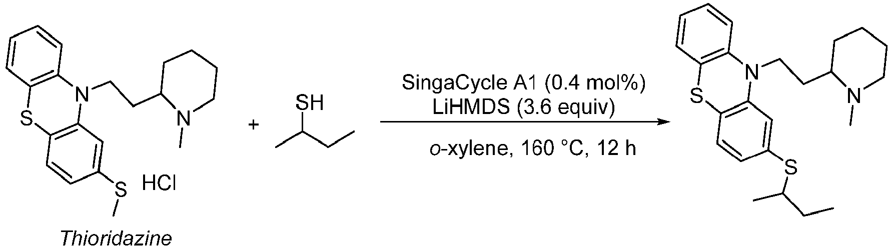

In the glovebox, thioridazine (0.2 mmol), sec-butyl thiol (2.0 equiv, 0.4 mmol), LiHMDS (3.6 equiv), and SingaCycle A1 (0.4 mol%) were added into an oven-dried 8 ml vial with a magnetic stirring bar, followed by addition of o-xylene (1.0 ml). The vial was sealed and removed out of the glovebox and heated to 160 °C. After 12 h, the vial was cooled to room temperature. The reaction was diluted with ethyl acetate and washed with NaOH solution. The aqueous phase was extracted with ethyl acetate 3 times. The collected organic phases were dried over anhydrous Na₂SO₄. The solvent was removed under reduced pressure and the residue was purified by flash column chromatography (0-5% MeOH in DCM) to give the title product. Isolated as orange oil (58.3 mg, 71%). ¹H NMR (500 MHz, Chloroform-d) δ 7.19-7.10 (m, 2H), 7.04 (d, *J* = 7.9 Hz, 1H), 6.96 (dd, *J* = 7.9, 1.7 Hz, 1H), 6.95-6.88 (m, 2H), 6.91-6.85 (m, 1H), 3.96 (ddd, *J =* 13.8, 8.5, 5.3 Hz, 1H), 3.85 (ddd, *J* = 13.8, 8.5, 6.3 Hz, 1H), 3.10 (q, *J* = 6.3 Hz, 1H), 2.88 (d, *J =* 11.3 Hz, 1H), 2.24 (s, 3H), 2.21-2.10 (m, 3H), 1.89 (d, *J* = 8.5 Hz, 1H), 1.78-1.69 (m, 2H), 1.67-1.58 (m, 3H), 1.58-1.47 (m, 2H), 1.36-1.26 (m, 1H), 1.25 (d, *J* = 6.3 Hz, 3H), 0.99 (t, *J* = 7.4 Hz, 3H). ¹³C NMR (126 MHz, CDCl₃) δ 145.4, 144.9, 134.4, 127.5, 127.43, 127.35, 126.3, 125.2, 124.3, 122.7, 119.6, 115.8, 62.2, 56.8, 45.5 (d, *J* = 1.8 Hz), 43.9, 42.7, 30.5, 29.6, 29.5, 25.3, 23.9, 20.5, 11.5. HRMS C₂₄H₃₂S₂N₂ [M+H]+; calculated 413.2079, found: 413.2083.

### Example 51

### 2-(((1s,3s)-adamantan-1-yl)thio)-10-(2-(1-methylpiperidin-2-yl)ethyl)-10H-phenothiazine

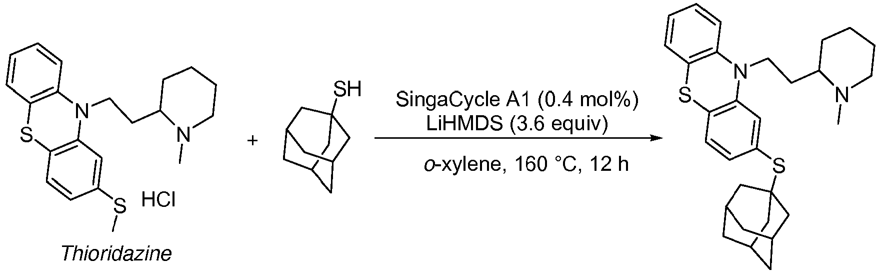

In the glovebox, thioridazine (0.2 mmol), 1-adamentanethiol (2.0 equiv, 0.4 mmol), LiHMDS (3.6 equiv), and SingaCycle A1 (0.4 mol%) were added into an oven-dried 8 ml vial with a magnetic stirring bar, followed by addition of *o*-xylene (1.0 ml). The vial was sealed and removed out of the glovebox and heated to 160 °C. After 12 h, the vial was cooled to room temperature. The reaction was diluted with ethyl acetate and washed with NaOH solution. The aqueous phase was extracted with ethyl acetate 3 times. The collected organic phases were dried over anhydrous Na₂SO₄. The solvent was removed under reduced pressure and the residue was purified by flash column chromatography (0-5% MeOH in DCM) to give the title product. Isolated as syrup like liquid (59.4 mg, 61%). ¹H NMR (500 MHz, Chloroform-d) δ 7.20-7.11 (m, 2H), 7.08-7.03 (m, 2H), 6.98 (d, *J =* 1.3 Hz, 1H), 6.97-6.87 (m, 2H), 3.98 (ddd, *J* = 13.6, 8.2, 5.2 Hz, 1H), 3.87 (ddd, *J* = 14.2, 8.2, 6.6 Hz, 1H), 3.00-2.82 (m, 1H), 2.52-2.18 (m, 6H), 2.07-1.98 (m, 3H), 1.97-1.86 (m, 1H), 1.80 (d, *J* = 2.8 Hz, 6H), 1.74-1.71 (m, 2H), 1.69-1.52 (m, 8H), 1.54-1.47 (m, 1H), 1.37-1.17 (m, 1H). ¹³C NMR (126 MHz, CDCl₃) δ 144.9, 131.7, 129.5, 127.6, 127.5, 127.4, 126.9, 126.6, 125.0, 124.4, 122.8, 115.8, 62.2, 56.7, 48.2, 43.9, 43.7, 42.5, 36.1, 30.2, 30.0, 29.4, 25.1, 23.7. HRMS C₃₀H₃₈S₂N₂ [M+H]+; calculated 491.2549, found: 491.2552.

### Example 52 - Procedure for depolymerization

### 1,4-bis(cyclopentylthio)benzene

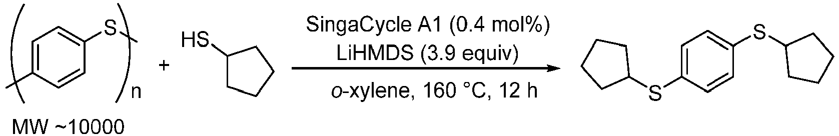

In the glovebox, poly(1,4-phenylene sulfide (MW-10000, 0.5 equiv, 0.1 mmol), 1-octanethiol (2.0 equiv, 0.4 mmol), LiHMDS (3.9 equiv), and SingaCycle A1 (0.4 mol%) were added into an oven-dried 8 ml vial with a magnetic stirring bar, followed by addition of *o*-xylene (2.0 ml). The vial was sealed and removed out of the glovebox and heated to 160 °C. After 12 h, the vial was cooled to room temperature. The reaction was diluted with ethyl acetate and washed with NaOH solution. The aqueous phase was extracted with ethyl acetate 3 times. The collected organic phases were dried over anhydrous Na₂SO₄. The solvent was removed under reduced pressure and the residue was purified by flash column chromatography (pentane/ethyl acetate 40:1) to give the title product. Isolated as a pale yellow liquid (24.5 mg, 89 %). ¹H NMR (500 MHz, Chloroform-d) δ 7.19 (d, *J* = 1.3 Hz, 4H), 3.54-3.43 (m, 2H), 2.03-1.92 (m, 4H), 1.79-1.66 (m, 4H), 1.61-1.48 (m, 8H). ¹³C NMR (126 MHz, CDCl₃) δ 134.9, 130.5, 46.2, 33.5, 24.8. HRMS C₁₆H₂₂S₂ [M]⁺; calculated 278.1163, found: 278.1166.

### Preparation Examples for the Nickel catalyzed C/S bond metathesis by arylation

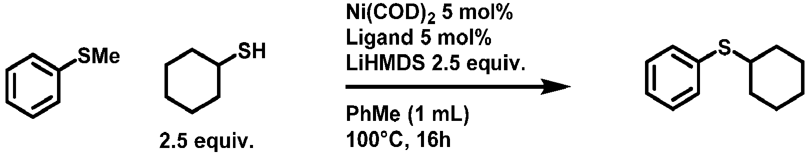

### General procedure

In a glovebox, an oven-dried 8 mL vial was charged with Bis-(dicyclohexylphosphino)-ethan (10.57 mg, 0.025 mmol, 5 mol%), Bis-(1,5-cyclooctadien)-nickel(0) (6.88 mg, 0.025 mmol, 5 mol%), Lithium bis(trimethylsilyl)amide (209.16 mg, 1.25 mmol, 2.5 equiv.), Methylthio arene (0.5 mmol, 1 equiv.), Alkylthiol (1.25 mmol, 2.5 equiv.) and toluene (1.25 mL, 0.4 mol/L). The reaction mixture was stirred at 100°C for 12h. The reaction mixture was allowed to cool to room temperature and was diluted with EtOAc and an aqueous solution of NaOH (1 mol/L). The layers were separated and the aqueous layer was extracted three times with EtOAc. The combined organic layers were dried over anhydrous MgSO₄ and concentrated to dryness. The residue was purified by FC (SiO₂, *n*-pentane to *n*-pentane:MTBE) to afford the title compound.

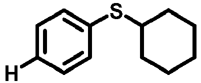

### Example 53

### Cyclohexyl(phenyl)sulfane (1a)

Following the general procedure using thioanisole (62.1 mg, 0.5 mmol) and cyclohexanethiol (145.3 mg, 1.25 mmol). Purification by FC (SiO₂, *n*-pentane to *n-*pentane:MTBE 100:1) to afford the title compound (85.6 mg, 89%) as a colorless liquid. ¹H NMR (501 MHz, Chloroform-d) δ 7.42 - 7.37 (m, 2H), 7.31 - 7.26 (m, 2H), 7.24 - 7.19 (m, 1H), 3.11 (tt, J = 10.5, 3.7 Hz, 1H), 2.03 - 1.95 (m, 2H), 1.82 - 1.72 (m, 2H), 1.62 (dddd, J = 11.6, 4.5, 2.7, 1.4 Hz, 1H), 1.41 - 1.21 (m, 5H). ¹³C NMR (126 MHz, Chloroform-d) δ 135.32, 132.00, 128.88, 126.71, 46.73, 33.50, 26.21, 25.92.

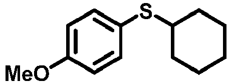

### Example 54

### Cyclohexyl(4-methoxyphenyl)sulfane (1b)

Following the general procedure using 4-(Methylmercapto)-anisol (77.1 mg, 0.5 mmol) and cyclohexanethiol (145.3 mg, 1.25 mmol). Purification by FC (SiO₂, *n-*pentane to n-pentane:MTBE 100:1) to afford the title compound (108.1 mg, 98%) as a slightly yellow oil. ¹H NMR (501 MHz, Chloroform-d) δ 7.42 - 7.35 (m, 2H), 6.89 - 6.80 (m, 2H), 3.80 (s, 3H), 2.90 (tt, J = 10.6, 3.7 Hz, 1H), 1.97 - 1.89 (m, 2H), 1.79 - 1.71 (m, 2H), 1.65 - 1.56 (m, 1H), 1.37 - 1.15 (m, 5H). ¹³C NMR (126 MHz, Chloroform-d) δ 159.44, 135.71, 125.14, 114.41, 55.44, 48.06, 33.52, 26.26, 25.92.

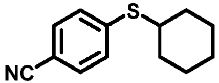

### Example 55

### 4-(Cyclohexylthio)benzonitrile

Following the general procedure using 4-(Methylthio)-benzonitrile (74.6 mg, 0.5 mmol) and cyclohexanethiol (145.28 mg, 1.25 mmol). Purification by FC (SiO₂, *n-*pentane to n-pentane:MTBE 20:1) to afford the title compound (64.4 mg, 59%) as a slightly yellow oil. ¹H NMR (501 MHz, Chloroform-d) δ 7.55 - 7.48 (m, 2H), 7.37 - 7.30 (m, 2H), 3.29 (tt, J = 10.3, 3.7 Hz, 1H), 2.07 - 1.96 (m, 2H), 1.84 - 1.76 (m, 2H), 1.66 (dddd, J = 12.9, 4.7, 3.0, 1.4 Hz, 1H), 1.50 - 1.23 (m, 5H). ¹³C NMR (126 MHz, Chloroform-d) δ 144.11, 132.33, 128.71, 119.05, 108.58, 45.06, 33.09, 26.01, 25.75.

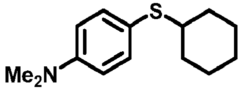

### Example 56

### 4-(Cyclohexylthio)-N,N-dimethylaniline

Following the general procedure using N,N-dimethyl-4-(methylthio)aniline (83.6 mg, 0.5 mmol) and cyclohexanethiol (145.28 mg, 1.25 mmol). Purification by FC (SiO₂, n-pentane to n-pentane:MTBE 50:1) to afford the title compound (111.8 mg, 95%) as an orange oil. ¹H NMR (501 MHz, Chloroform-*d*) δ 7.38 - 7.30 (m, 2H), 6.69 - 6.61 (m, 2H), 2.96 (s, 6H), 2.82 (tt, *J* = 10.7, 3.7 Hz, 1H), 2.01 - 1.89 (m, 2H), 1.74 (dt, *J =* 11.9, 4.1 Hz, 2H), 1.62 - 1.53 (m, 1H), 1.40 - 1.14 (m, 5H). ¹³C NMR (126 MHz, Chloroform-*d*) δ 150.27, 136.18, 119.40, 112.61, 48.35, 40.54, 33.57, 33.39, 26.33, 25.96.

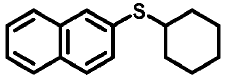

### Example 57

### Cyclohexyl(naphthalen-2-yl)sulfane

Following the general procedure using 2-(Methylthio)-naphthalin (87.1 mg, 0.5 mmol) and cyclohexanethiol (145.3 mg, 1.25 mmol). Purification by FC (SiO₂, *n-*pentane to n-pentane:MTBE 100:1) to afford the title compound (114.9 mg, 95%) as a colorless oil. ¹H NMR (501 MHz, Chloroform-*d*) δ 7.86 (d, *J* = 1.7 Hz, 1H), 7.83 - 7.71 (m, 3H), 7.51 - 7.36 (m, 3H), 3.24 (tt, *J* = 10.6, 3.7 Hz, 1H), 2.08 - 1.99 (m, 2H), 1.84 - 1.74 (m, 2H), 1.63 (dddd, *J* = 12.1, 4.7, 2.9, 1.4 Hz, 1H), 1.49 - 1.21 (m, 5H). ¹³C NMR (126 MHz, Chloroform-*d*) δ 133.82, 132.80, 132.22, 130.32, 129.74, 128.34, 127.80, 127.40, 126.53, 125.97, 46.73, 33.52, 29.86, 26.21, 25.93.

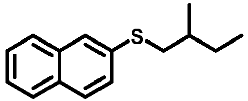

### Example 58

### (2-Methylbutyl)(naphthalen-2-yl)sulfane

Following the general procedure using 2-(Methylthio)-naphthalin (87.1 mg, 0.5 mmol) and 2-Methyl-1-butanthiol (130.3 mg, 1.25 mmol). Purification by FC (SiO₂, n-pentane to n-pentane:MTBE 50:1) to afford the title compound (112.9 mg, 98%) as a slightly brown oil. ¹H NMR (501 MHz, Chloroform-*d*) δ 7.81 - 7.70 (m, 4H), 7.47 (ddd, *J* = 8.1, 6.7, 1.4 Hz, 1H), 7.46 - 7.38 (m, 2H), 3.07 (dd, *J* = 12.5, 5.8 Hz, 1H), 2.86 (dd, *J* = 12.5, 7.5 Hz, 1H), 1.79 - 1.67 (m, 1H), 1.59 (dqd, *J* = 12.9, 7.4, 5.3 Hz, 1H), 1.38 - 1.25 (m, 1H), 1.07 (d, *J* = 6.7 Hz, 3H), 0.94 (t, *J* = 7.4 Hz, 3H). ¹³C NMR (126 MHz, Chloroform-*d*) δ 135.25, 133.95, 131.69, 128.36, 127.83, 127.36, 127.08, 126.60, 126.25, 125.53, 40.70, 34.66, 29.86, 29.00, 19.15, 11.44.

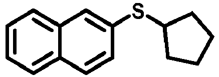

### Example 59

### Cyclopentyl(naphthalen-2-yl)sulfane

Following the general procedure using 2-(Methylthio)-naphthalin (87.1 mg, 0.5 mmol) and Cyclopentanthiol (127.8 mg, 1.25 mmol). Purification by FC (SiO₂, *n-*pentane to n-pentane:MTBE 100:1) to afford the title compound (98.7 mg, 86%) as a slightly yellow oil. ¹H NMR (501 MHz, Chloroform-*d*) δ 7.82 - 7.71 (m, 4H), 7.51 - 7.36 (m, 3H), 3.73 (tt, *J* = 7.2, 6.0 Hz, 1H), 2.17 - 2.04 (m, 2H), 1.89 - 1.75 (m, 2H), 1.75 - 1.58 (m, 3H). ¹³C NMR (126 MHz, Chloroform-*d*) δ 135.01, 133.91, 131.84, 128.29, 128.20, 127.82, 127.23, 126.55, 125.66, 45.94, 33.72, 29.86, 25.02.

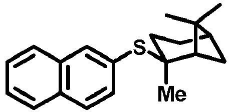

### Example 60

### Naphthalen-2-yl((1S,2S,5R)-2,6,6-trimethylbicyclo[3.1.1]heptan-2-yl)sulfane

Following the general procedure using 2-(Methylthio)-naphthalin (87.1 mg, 0.5 mmol) and 2-,3-,10-Mercaptopinane (212.9 mg, 1.25 mmol). Purification by FC (SiO₂, n-pentane to n-pentane:MTBE 100:1) to afford the title compound (134.9 mg, 91%) as a pink oil. ¹H NMR (501 MHz, Chloroform-*d*) δ 7.78 (dd, *J* = 7.9, 1.3 Hz, 1H), 7.77 - 7.68 (m, 3H), 7.46 (ddd, *J* = 8.2, 6.8, 1.4 Hz, 1H), 7.42 (ddd, *J =* 8.3, 6.2, 1.6 Hz, 2H), 3.18 - 3.02 (m, 2H), 2.99 - 2.90 (m, 0.34H), 2.40 - 2.23 (m, 2H), 2.15 - 1.74 (m, 5H), 1.64 (dddd, *J =* 15.1, 10.8, 6.8, 5.6 Hz, 1H), 1.54 (s, 0.20H), 1.51 - 1.36 (m, 0.37H), 1.29 (s, 0.17H), 1.22 (d, *J* = 11.4 Hz, 3H), 1.06 (s, 2H), 0.88 (d, *J* = 9.7 Hz, 1H), 0.80 (s, 0.52H). ¹³C NMR (126 MHz, Chloroform-*d*) δ 135.30, 134.91, 133.97, 133.95, 131.73, 131.68, 128.38, 128.33, 127.84, 127.43, 127.33, 127.10, 127.08, 126.59, 126.43, 126.11, 125.55, 125.51, 124.77, 45.78, 45.27, 41.42, 41.01, 40.81, 40.69, 40.05, 39.69, 39.59, 38.87, 34.86, 33.46, 29.86, 28.10, 27.71, 26.85, 26.39, 26.30, 24.45, 23.60, 23.47, 22.42, 22.27, 20.25, 19.83.

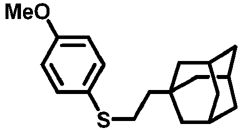

### Example 61

### (2-(Adamantan-1-yl)ethyl)(4-methoxyphenyl)sulfane

Following the general procedure using 4-(Methylmercapto)-anisol (77.1 mg, 0.5 mmol) and 2-(1-Adamantyl)-ethanthiol (245.4 mg, 1.25 mmol). Purification by FC (SiO₂, n-pentane to n-pentane:MTBE 50:1) to afford the title compound (146.7 mg, 97%) as a pink oil. ¹H NMR (501 MHz, Chloroform-*d*) δ 7.35 - 7.26 (m, 2H), 6.88 - 6.81 (m, 2H), 3.80 (s, 3H), 2.84 - 2.76 (m, 2H), 1.94 (p, *J* = 3.1 Hz, 3H), 1.73 - 1.57 (m, 6H), 1.47 (d, *J =* 2.8 Hz, 6H), 1.41 - 1.34 (m, 2H). ¹³C NMR (126 MHz, Chloroform-*d*) δ 158.73, 132.56, 127.27, 114.64, 55.48, 44.11, 42.35, 37.24, 32.88, 29.89, 28.77.

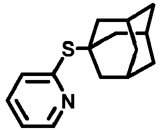

### Example 62

### 2-(Adamantan-1-ylthio)pyridine

Following the general procedure using 2-(Methylthio)-pyridin (62.6 mg, 0.5 mmol) and Tricyclo[3.3.1.13,7]decan-1-thiol (210.4 mg, 1.25 mmol). Purification by FC (SiO₂, n-pentane to n-pentane:MTBE 50:1) to afford the title compound (105.7 mg, 96%) as a beige solid. ¹H NMR (501 MHz, Chloroform-*d*) δ 8.53 (ddd, *J* = 4.9, 2.0, 0.9 Hz, 1H), 7.53 (td, *J* = 7.7, 2.0 Hz, 1H), 7.38 (dt, *J* = 7.9, 1.1 Hz, 1H), 7.10 (ddd, *J =* 7.4, 4.8, 1.1 Hz, 1H), 2.10 - 2.02 (m, 9H), 1.69 (t, *J =* 3.0 Hz, 6H). ¹³C NMR (126 MHz, Chloroform-*d*) δ 156.97, 149.73, 136.11, 129.11, 121.44, 50.19, 43.77, 36.43, 30.24.

**Ligands investigation**

| Ligand | Yield (%)^{a,b} |
|---|---|
| 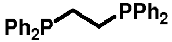 | 46 |
| 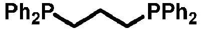 | 51 |
| 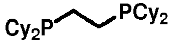 | 88 |
| 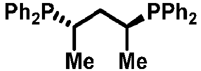 | 75 |
| 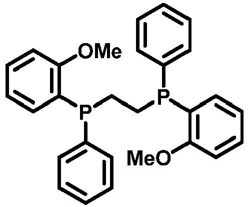 | 81 |

| | |
|---|---|
| (a) Reactions were performed on 0.2 mmol scale of thioanisole. (b) Product yield was determined by GC analysis using dodecane as internal standard. | |

## Claims

1. A process for a catalytic aryl transfer wherein an aryl-compound (I) is reacted with an hydrocarbon (II) in the presence of a Pd- or Ni- catalyst coordinated by electron rich ligands and in the presence of a base in an organic solvent, as represented in the following reaction scheme: 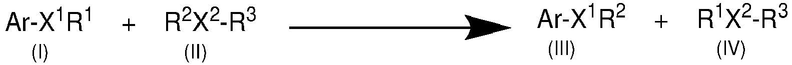 wherein
Ar is aryl, heteroaryl or vinyl, each being optionally substituted by one or more groups selected from straight chain or branched chain alkyl, cycloalkyl, heterocycloalkyl, alkenyl, alkynyl, aryl, aralkyl, heteroaryl, heteroaralkyl, ether, acetal, silyl ether or amine, or by a heterosubstituent;
X¹ and X² may be the same or different and are each S or Se, preferably S,
R¹ is H or methyl, a straight chain or branched C₂-C₁₆-alkyl or aryl, each optionally being substituted by one or more groups selected from straight chain or branched chain alkyl, cycloalkyl, heterocycloalkyl, alkenyl, alkynyl, aryl, aralkyl, heteroaryl, heteroaralkyl, ether, acetal, silyl ether or amine, or by a heterosubstituent;
R² is an primary, secondary or tertiary alkyl hydrocarbon or aryl hydrocarbon, each being optionally being substituted by one or more groups selected from straight chain or branched chain alkyl, cycloalkyl, heterocycloalkyl, alkenyl, alkynyl, aryl, aralkyl, heteroaryl, heteroaralkyl, or by a heterosubstituent;
R³ is H,
the Pd- or Ni-catalyst coordinated by electron rich ligands is selected from the group consisting of Pd(OAc)₂, Pd₂(dba)₃, PdCl₂, PdCl₂(MeCN)₂, Ni(COD)₂.
the base is selected from the group consisting of LiHMDS, KHMDS, NaHDMS, LiO*t*Bu, KO*t*Bu, NaO*t*Bu.
the electron rich ligands may be the same or different and are selected from the group consisting of IPENT, SIPr, Icy and IPr.

2. Process according to claim 1, wherein the catalyst is a Pd-NHC complex, preferably a [(NHC)Pd(dimethylbenzylamine)CI] complex.

3. Process according to claim 1, wherein the catalyst is a Ni-bisphosphine complex, preferably generated from a mixture of Ni(COD)₂ and Bis-(dicyclohexylphosphino)-ethan.

4. Process according to claim 1 or 2, wherein the catalyst is present in an amount in the range of 0.05 mol% to 1 mol% of the aryl-compound (I), preferably in the range of 0.2 mol% to 0.6 mol% of the aryl-compound (I).

5. Process according to any one of claims 1 to 3, wherein the base is a lithium base, sodium base or potassium base, preferably a lithium base, more preferably LiHMDS.

6. Process according to any one of claims 1 to 4, wherein the base is present in an amount in the range of 1 equivalent to 6 equivalents, preferably in the range of 1.5 equivalents to 4 equivalents of the reaction partners.

7. Process according to any one of claims 1 to 5, wherein the aryl-compound (I) is reacted with the hydrocarbon (II) at a temperature in the range of 25°C to 250°C, preferably in the range of 80°C to 200°C, for 4 h to 20 h, preferably 8 h to 16 h.

8. Process according to any one of claims 1 to 6, wherein the aryl-compound (I) is reacted with the hydrocarbon (II) in an aromatic solvent or an aliphatic hydrocarbon solvent, more preferably in toluene, benzene, xylene, cumene, chlorobenzene or dichlorobenzene.

9. Process according to any one of claims 1 to 7, wherein Ar is phenyl, 4-methylphenyl or naphtyl, each optionally being substituted by one or more groups selected from straight chain or branched chain alkyl, ether, acetal, silyl ether or amine, preferably being substituted by methyl, ketals, methoxy, MOMO, TIPSO, OCF₃, OBn, CF₃, F, TMS, NMe₂, CN, pyridyl, pyrazinyl, benzothiazyl, phenylvinyl, t-butyl or 5-phenyl- benzothiazyl.

10. Process according to any one of claims 1 to 8, wherein Ar or Ar-X is a component of a polymer.

11. Process according to any one of claims 1 to 9, wherein X is S.

12. Process according to any one of claims 1 to 10, wherein R1 is H, methyl, phenyl or 4-methoxyphenyl.

13. Process according to any one of claims 1 to 11, wherein R² is cyclohexyl, cyclopentyl, 2-methylbutyl, 1-methyl-propyl, nC₁₂H₂₅, adamantyl, 2-phenylethyl, 1-methyl-5-dimethyl-bicyclo[4.1.0]heptyl-, nC₈H₁₇, benzyl, optionally substituted steroid residue, 2-amantadyl-ethyl or C₈H₁₇.

14. Process according to any one of claims 1 to 13, wherein the hydrocarbon (II) is present an amount in the range of 0.5 equivalents to 6 equivalents of the aryl-compound (I), preferably in the range of 1.5 equivalent to 4 equivalents of the aryl-compound (I).

## Patentansprüche

1. Verfahren zum katalytischen Aryltransfer, bei dem eine Arylverbindung (I) mit einem Kohlenwasserstoff (II) in Gegenwart eines durch elektronenreiche Liganden koordinierten Pd- oder Ni-Katalysators und in Gegenwart einer Base in einem organischen Lösungsmittel umgesetzt wird,
wie im folgenden Reaktionsschema dargestellt: 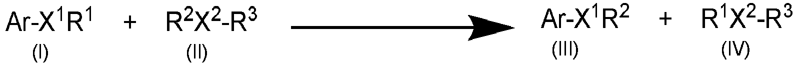 wobei
Ar Aryl, Heteroaryl oder Vinyl ist, wobei jedes gegebenenfalls durch eine oder mehrere Gruppen, ausgewählt aus geradkettigem oder verzweigtkettigem Alkyl, Cycloalkyl, Heterocycloalkyl, Alkenyl, Alkinyl, Aryl, Aralkyl, Heteroaryl, Heteroaralkyl, Ether, Acetal, Silylether oder Amin, oder durch einen Heterosubstituenten substituiert ist;
X¹ und X² gleich oder verschieden sein können und jeweils S oder Se, vorzugsweise S sind,
R¹ ist H oder Methyl, ein geradkettiges oder verzweigtes C₂-C₁₆-Alkyl oder Aryl, jeweils gegebenenfalls substituiert durch eine oder mehrere Gruppen, ausgewählt aus geradkettigem oder verzweigtem Alkyl, Cycloalkyl, Heterocycloalkyl, Alkenyl, Alkinyl, Aryl, Aralkyl, Heteroaryl, Heteroaralkyl, Ether, Acetal, Silylether oder Amin, oder durch einen Heterosubstituenten;
R² ein primärer, sekundärer oder tertiärer Alkylkohlenwasserstoff oder Arylkohlenwasserstoff ist, wobei jeder gegebenenfalls durch eine oder mehrere Gruppen, ausgewählt aus geradkettigem oder verzweigtkettigem Alkyl, Cycloalkyl, Heterocycloalkyl, Alkenyl, Alkinyl, Aryl, Aralkyl, Heteroaryl, Heteroaralkyl, oder durch einen Heterosubstituenten substituiert ist;
R³ H ist,
der Pd- oder Ni-Katalysator, koordiniert durch elektronenreiche Liganden, ausgewählt ist aus der Gruppe bestehend aus Pd(OAc)₂, Pd2(dba)₃, PdCl₂, PdCl₂(MeCN)₂, Ni(COD)₂.
die Base ausgewählt ist aus der Gruppe bestehend aus LiHMDS, KHMDS, NaHDMS, LiOtBu, KOtBu, NaOtBu.
die elektronenreichen Liganden gleich oder verschieden sein können und ausgewählt sind aus der Gruppe bestehend aus IPENT, SIPr, Icy und IPr.

2. Verfahren nach Anspruch 1, wobei der Katalysator ein Pd-NHC-Komplex, vorzugsweise ein [(NHC)Pd(Dimethylbenzylamin)Cl]-Komplex ist.

3. Verfahren nach Anspruch 1, **dadurch gekennzeichnet, dass** der Katalysator ein Ni-Bisphosphin-Komplex ist, der vorzugsweise aus einer Mischung von Ni(COD)₂ und Bis-(dicyclohexylphosphino)-ethan erzeugt wird.

4. Verfahren nach Anspruch 1 oder 2, **dadurch gekennzeichnet, dass** der Katalysator in einer Menge im Bereich von 0,05 mol% bis 1 mol% der Arylverbindung (I), vorzugsweise im Bereich von 0,2 mol% bis 0,6 mol% der Arylverbindung (I) vorliegt.

5. Verfahren nach einem der Ansprüche 1 bis 3, **dadurch gekennzeichnet, dass** die Base eine Lithiumbase, Natriumbase oder Kaliumbase, vorzugsweise eine Lithiumbase, besonders bevorzugt LiHMDS ist.

6. Verfahren nach einem der Ansprüche 1 bis 4, wobei die Base in einer Menge im Bereich von 1 Äquivalent bis 6 Äquivalenten, vorzugsweise im Bereich von 1,5 Äquivalenten bis 4 Äquivalenten der Reaktionspartner vorliegt.

7. Verfahren nach einem der Ansprüche 1 bis 5, **dadurch gekennzeichnet, dass** die Arylverbindung (I) mit dem Kohlenwasserstoff (II) bei einer Temperatur im Bereich von 25°C bis 250°C, vorzugsweise im Bereich von 80°C bis 200°C, für 4 h bis 20 h, vorzugsweise 8 h bis 16 h, umgesetzt wird.

8. Verfahren nach einem der Ansprüche 1 bis 6, **dadurch gekennzeichnet, dass** die Arylverbindung (I) mit dem Kohlenwasserstoff (II) in einem aromatischen Lösungsmittel oder einem aliphatischen Kohlenwasserstofflösungsmittel, besonders bevorzugt in Toluol, Benzol, Xylol, Cumol, Chlorbenzol oder Dichlorbenzol umgesetzt wird.

9. Verfahren nach einem der Ansprüche 1 bis 7, wobei Ar Phenyl, 4-Methylphenyl oder Naphthyl ist, die jeweils gegebenenfalls durch eine oder mehrere Gruppen substituiert sind, die ausgewählt sind aus geradkettigem oder verzweigtem Alkyl, Ether, Acetal, Silylether oder Amin substituiert ist, vorzugsweise durch Methyl, Ketale, Methoxy, MOMO, TIPSO, OCF₃, OBn, CF₃, F, TMS, NMe₂, CN, Pyridyl, Pyrazinyl, Benzothiazyl, Phenylvinyl, t-Butyl oder 5-Phenyl-benzothiazyl substituiert ist.

10. Verfahren nach einem der Ansprüche 1 bis 8, wobei Ar oder Ar-X ein Bestandteil eines Polymers ist.

11. Verfahren nach einem der Ansprüche 1 bis 9, wobei X S ist.

12. Verfahren nach einem der Ansprüche 1 bis 10, wobei R¹ H, Methyl, Phenyl oder 4-Methoxyphenyl ist.

13. Verfahren nach einem der Ansprüche 1 bis 11, wobei R² Cyclohexyl, Cyclopentyl, 2-Methylbutyl, 1-Methylpropyl, nC₁₂H₂₅, Adamantyl, 2-Phenylethyl, 1-Methyl-5-dimethyl-bicyclo[4.1.0]heptyl-, nC₈H₁₇, Benzyl, gegebenenfalls substituierter Steroidrest, 2-Amantadyl-ethyl oder C₈H₁₇ ist.

14. Verfahren nach einem der Ansprüche 1 bis 13, wobei der Kohlenwasserstoff (II) in einer Menge im Bereich von 0,5 Äquivalenten bis 6 Äquivalenten der Arylverbindung (I), vorzugsweise im Bereich von 1,5 Äquivalenten bis 4 Äquivalenten der Arylverbindung (I) vorliegt.

## Revendications

1. Procédé de transfert d'aryle catalytique dans lequel un composé aryle (I) est mis à réagir avec un hydrocarbure (II) en présence d'un catalyseur Pd- ou Ni-coordonné par des ligands riches en électrons en présence d'une base dans un solvant organique, tel que représenté dans le schéma réactionnel suivant : 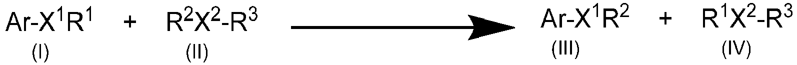 dans lequel
Ar est un groupe aryle, hétéroaryle ou vinyle, chacun étant éventuellement substitué par un ou plusieurs groupes choisis parmi des groupes alkyle à chaîne linéaire ou à chaîne ramifiée, cycloalkyle, hétérocycloalkyle, alcényle, alcynyle, aryle, aralkyle, hétéroaryle, hétéroaralkyle, éther, acétal, silyl éther ou amine, ou par un hétérosubstituant ;
X¹ et X² peuvent être identiques ou différents et sont chacun S ou Se, de préférence S,
R¹ est H ou un groupe méthyle, alkyle en C₂ à C₁₆ à chaîne linéaire ou ramifié ou aryle, chacun étant éventuellement substitué par un ou plusieurs groupes choisis parmi des groupes alkyle à chaîne linéaire ou à chaîne ramifiée, cycloalkyle, hétérocycloalkyle, alcényle, alcynyle, aryle, aralkyle, hétéroaryle, hétéroaralkyle, éther, acétal, silyl éther ou amine, ou par un hétérosubstituant;
R² est un groupe hydrocarbure alkyle primaire, secondaire ou tertiaire ou hydrocarbure aryle, chacun étant éventuellement substitué par un ou plusieurs groupes choisis des groupes alkyle à chaîne linéaire ou à chaîne ramifiée, cycloalkyle, hétérocycloalkyle, alcényle, alcynyle, aryle, aralkyle, hétéroaryle, hétéroaralkyle, ou par un hétérosubstituant;
R³ est H,
le catalyseur Pd- ou Ni- coordonné par des ligands riches en électrons est choisi dans le groupe constitué par Pd(OAc)₂, Pd₂(dba)₃, PdCl₂, PdCl₂(MeCN)₂, Ni(COD)₂,
la base est choisie dans le groupe constitué par LiHMDS, KHMDS, NaHDMS, LiO*t*Bu, KO*t*Bu, NaO*t*Bu,
les ligands riches en électrons peuvent être identiques ou différents et sont choisis dans le groupe constitué par IPENT, SIPr, Icy et IPr.

2. Procédé selon la revendication 1, dans lequel le catalyseur est un complexe Pd-NHC, de préférence un complexe [(NHC)Pd(diméthylbenzylamine)Cl].

3. Procédé selon la revendication 1, dans lequel le catalyseur est un complexe Ni-bisphosphine, de préférence produit à partir d'un mélange de Ni(COD)₂ et de bis(dicyclohexylphosphino)-éthane.

4. Procédé selon la revendication 1 ou 2, dans lequel le catalyseur est présent en une quantité dans la plage de 0,05 % en mole à 1 % en mole du composé aryle (I), de préférence dans la plage de 0,2 % en mole à 0,6 % en mole du composé aryle (I).

5. Procédé selon l'une quelconque des revendications 1 à 3, dans lequel la base est une base de lithium, une base de sodium ou une base de potassium, de préférence une base de lithium davantage de préférence LiHMDS.

6. Procédé selon l'une quelconque des revendications 1 à 4, dans lequel la base est présente en une quantité dans la plage de 1 équivalent à 6 équivalents, de préférence dans la plage de 1,5 équivalents à 4 équivalents des partenaires réactionnels.

7. Procédé selon l'une quelconque des revendications 1 à 5, dans lequel le composé aryle (I) est mis à réagir avec l'hydrocarbure (II) à une température dans la plage de 25 °C à 250 °C, de préférence dans la plage de 80 °C à 200 °C, durant 4 h à 20 h, de préférence 8 h à 16 h.

8. Procédé selon l'une quelconque des revendications 1 à 6, dans lequel le composé aryle (I) est mis à réagir avec l'hydrocarbure (II) dans un solvant aromatique ou un solvant hydrocarbure aliphatique, davantage de préférence dans le toluène, le benzène, le xylène, le cumène, le chlorobenzène ou le dichlorobenzène.

9. Procédé selon l'une quelconque des revendications 1 à 7, dans lequel Ar est un groupe phényle, 4-méthylphényle ou naphtyle, chacun étant éventuellement substitué par un ou plusieurs groupes choisis parmi des groupes alkyle à chaîne linéaire ou à chaîne ramifiée, éther, acétal, silyl éther ou amine, de préférence étant substitués par des groupes méthyle, cétals, méthoxy, MOMO, TIPSO, OCF₃, OBn, CF₃, F, TMS, NMe₂, CN, pyridyle, pyrazinyle, benzothiazyle, phénylvinyle, t-butyle ou 5-phényl-benzothiazyle.

10. Procédé selon l'une quelconque des revendications 1 à 8, dans lequel Ar ou Ar-X est un composant d'un polymère.

11. Procédé selon l'une quelconque des revendications 1 à 9, dans lequel X est S.

12. Procédé selon l'une quelconque des revendications 1 à 10, dans lequel R1 est H, un groupe méthyle, phényle ou 4-méthoxyphényle.

13. Procédé selon l'une quelconque des revendications 1 à 11, dans lequel R² est un groupe cyclohexyle, cyclopentyle, 2-méthylbutyle, 1-méthyl-propyle, nC₁₂H₂₅, adamantyle, 2-phényléthyle, 1-méthyl-5-diméthyl-bicyclo[4.1.0]heptyle-, nC₈H₁₇, benzyle, éventuellement substitué par un résidu stéroïde, un groupe 2-amantadyl-éthyle ou C₈H₁₇.

14. Procédé selon l'une quelconque des revendications 1 à 13, dans lequel l'hydrocarbure (II) est présent en une quantité dans la plage de 0,5 équivalent à 6 équivalents du composé aryle (I), de préférence dans la plage de 1,5 équivalents à 4 équivalents du composé aryle (i).
